(19) Europäisches Patentamt
European Patent Office
Office européen des brevets

(11) EP 1 908 751 A1

(12) EUROPEAN PATENT APPLICATION

(43) Date of publication:
**09.04.2008 Bulletin 2008/15**

(21) Application number: **06425676.1**

(22) Date of filing: **03.10.2006**

(51) Int Cl.:
*C07D 213/74* (2006.01) *C07D 237/08* (2006.01)
*C07D 333/28* (2006.01) *C07D 333/24* (2006.01)
*A61K 31/44* (2006.01) *A61K 31/38* (2006.01)
*A61K 31/50* (2006.01) *A61P 35/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **EOS S.p.A.**
**20121 Milano (IT)**

(72) Inventor: **Spinelli, Silvano**
**20052 Monza (IT)**

(74) Representative: **Minoja, Fabrizio**
**Bianchetti Bracco Minoja S.r.l.**
**Via Plinio, 63**
**20129 Milano (IT)**

(54) **N-hydroxy benzamides with antitumour activity**

(57) The present invention relates to N-hydroxy benzamides of formula (**I**)

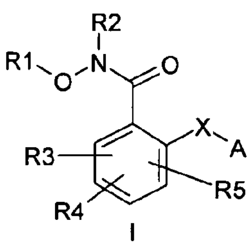

I

wherein groups R1-R5, X and A are as defined in the description, their preparation and their use as antitumour agents.

EP 1 908 751 A1

## Description

### Field of the invention

Object of the present invention are novel N-hydroxybenzamides useful for the treatment of pathologies characterised by abnormal cell proliferation, such as tumours. A further object of the present invention is the use of said N-hydroxybenzamides for the treatment of hyperproliferative pathologies in mammals, in particular in humans, and pharmaceutical compositions containing said compounds.

### Background of the invention

Eukaryotic cells respond to exogenous stimuli activating a series of signal transduction cascades having cellular effects like proliferation, differentiation or apoptosis. Further to interaction of growth factors or cytokines, the transmission of the stimulating signals from receptors to the nucleus requires the regulation of a kinase family known as MAPKs (mitogen activated protein kinases) or ERKs (extracellular signal regulated kinases). For example, the ability of growth factors to promote proliferation depends on the activation of tyrosine-kinase receptors which recruit proteins of the Ras family and sequentially activate Raf (MAPK kinase kinase), MEK (MAPK kinase) and ERK (MAPK). The MAP kinase cascade plays an important role in directing the signals from different extra-cellular stimuli and protooncogenes towards the nucleus, where the activation of specific transcription factors results in cellular responses like proliferation, differentiation, survival and apoptosis.

The transduction signal cascade that involves Raf/MEK/ERK is one of the most important and best known as far as human tumours are concerned. Studies on tumour lines and biopsies from human tumours demonstrated that the ERK cascade is constitutively activated in lung, colon, pancreas, kidney and ovary tumours (Hoshino et al, Oncogene 1999, 18, 813-822). It has been demonstrated that the activation of the Raf/MEK/ERK cascade is a necessary and sufficient condition for cell transformation (Mansour et al. Science, 1994, v. 265, pp. 966-970; Cowley et al, Cell, 1994, v. 77, pp. 841-852; Brunet et al., Oncogene, 1994, v. 9, pp. 3379-3387). Moreover, oncogenic forms of Ras are present in about one third of human tumours, among them 50% of colon tumours, 30% of lung tumours and more than 9 out of 10 pancreas tumours (Bos, J.L. Cancer Res. 1989, 49(17), 4682-4689). 60% of malignant melanomas and 40-70% of thyroid papillary tumours bear B-raf mutations (Davies, H et al., Nature 2002, 417(6892), 949-954; Cohen, Y et al, Cancer Res. 2003, 63(15), 4561-4567). The functional consequence of these mutations is a constitutively activated cascade of ERK kinase. MEK plays a pivotal role in this intracellular signals cascade and catalyzes the phosphorylation of ERK1 and ERK2 MAP kinases (Anderson et al., Nature 1990, v.343, pp. 651-653) which are the only MEK substrates identified so far (Seger et al., J. Biol. Chem., 1992, v. 267, pp. 14373-14381). Several studies demonstrated that MEK inhibition has potential therapeutic benefits. In particular, some MEK inhibitors arrest the growth of human tumours in experimental models (Sebolt-Leopold et al,, Nat. Med. 1999, 5, 810-816). Some MEK inhibitors, such as ARRY-142886 (Yeh, T. et al., Proc. Am. Assoc. Cancer Res. 2004, 45, Abst. 3888; Yeh, T et al., Proc. Am. Assoc. Cancer Res. 2004, 45, Abst. 3889; Lee, P. et al., Proc. Am. Assoc. Cancer Res. 2004, 45, Abst. 3890; Wallace, E. et al, Proceedings of the American Association of Cancer Research 2004, 45, Abs 3891; Winkler, J.D. et al., 16th EORTC-NCI-AACR Symposium on Molecular Targets and Cancer Therapeutics, Geneva, Switzerland, Sept 27-Oct 1, 2004, Abst. 342), PD-0325901 (Tecle, H et al., 29th American Chemical Society Medicinal Chemistry Symposium, Madison, WI, June 27-July 1, 2004; Kaufman, M. D. et al., Proc. Am. Assoc. Cancer Res. 2004, 45, Abst. 2477) and CI-1040 (Waterhouse et al., Proceedings of the American Society for Clinical Oncology 2003, 22, Abs 816) have entered clinical studies as antitumour agents. However, CI-1040, the first MEK inhibitor to enter clinical trials, showed no efficacy in phase II studies, where responses were not observed and only in few patients stabilisation of the pathological conditions was attained. The absence of clinical activity of CI-1040 is probably due to its low solubility, high metabolic clearance and low bioavailability. There is therefore the need to develop new MEK inhibitors with an improved pharmacological profile.

MEK inhibitors and their use in therapy have been disclosed in a number of patents and patent applications, for example US 5,525,625, WO 98/37881, WO 99/01421, WO 99/01426, WO 00/40235, WO 00/40237, WO 00/41505, WO 00/41994, WO 00/42002, WO 00/42003, WO 00/42022, WO 00/42029, WO 00/56706, WO 00/68199, WO 00/68200, WO 00/68201, WO 01/05390, WO 01/05391, WO 01/05392, WO 01/05393, WO 01/68619, WO 01/76570, WO 02/06213, WO 02/06520, WO 02/069960, WO 03/035626, WO 03/047523, WO 03/053960, WO 03/077855, WO 03/077914, WO 03/103717, WO 2004/005284, WO 2004/045617, WO 2004/056789, WO 2005/000818, WO 2005/007616, WO 2005/009975, WO 2005/011466, WO 2005/023759, WO 2005/023251, WO 2005/028426, WO 2005/046665, WO 2005/051300, WO 2005/051301, WO 2005/051302, WO 2005/051906, WO 2006/056427 e WO 2006/061712.

### Description of the invention

The present invention provides novel N-hydroxybenzamides, enantiomers and diastereoisomers thereof, as

well as pharmaceutically acceptable salts, pro-drugs and solvates able to inhibit MEK kinase and therefore useful for the treatment of tumours.

The compounds of the invention have the following general formula I:

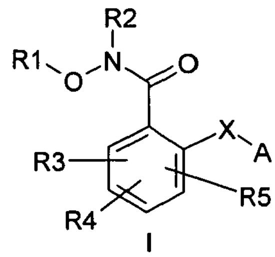

I

wherein

R1 is selected from hydrogen, trifluoromethyl, $C_{1-10}$ alkyl, $C_{2-10}$ alkenyl, $C_{2-10}$ alkynyl, $C_{3-10}$ cycloalkyl, $C_{3-10}$ cycloalkylalkyl, aryl, aralkyl, heteroaryl, heteroarylalkyl, heterocyclyl, heterocycloalkyl, said alkyl, alkenyl, alkynyl, cycloalkyl, aryl, heteroaryl and heterocyclyl groups being optionally susbstituted with one to three substituents independently selected from hydroxy, amino, alkyl or cycloalkyl;

R2 is selected from hydrogen, $C_{1-8}$ alkyl, CO-$C_{1-8}$ alkyl, aryl, aralkyl, or C3-10 cycloalkyl, wherein the alkyl, alkylcarbonyl, aryl, aralkyl or cycloalkyl groups are optionally substituted with one to three substituents independently selected from hydroxy, amino, alkyl or cycloalkyl;

R3, R4 and R5 are independently selected from hydrogen, hydroxy, halogen, trifluoromethyl, $C_{1-8}$ alkyl, $C_{1-8}$ alkoxy, nitro, cyano or $-(O)_m-CH_2)_n$-R6 and $-(NH)_m-CH_2)_n$-R6 groups, wherein m is 0 or 1, R6 is hydrogen, hydroxy, carboxy or NRaRb wherein Ra and Rb are independently selected from hydrogen and $C_{1-4}$ alkyl or, together with the nitrogen atom they are bound to, form a pyrrolidine, piperidine, morpholine or piperazine ring; X is selected from O, S, SO, $SO_2$, NH, $CH_2$, CO;

A is an aromatic or aliphatic 5 to 6-membered heterocycle, containing one to three nitrogen, oxygen or sulphur atoms and optionally substituted on the carbon atoms with 1, 2 or 3 R7 groups or, on the nitrogen atoms, with $C_{1-10}$ alkyl, aryl, arylalkyl groups or with oxygen atoms to give N-oxides;

R7 is independently selected from hydrogen, $C_{1-20}$ alkyl, $C_{2-20}$ alkenyl, $C_{2-20}$ alkynyl, -OR7a, -SR7a, -CN, nitro, azido, halogen, haloalkyl, $-NH_2$, - NH($C_{1-10}$ alkyl), -N($C_{1-10}$ alkyl)$_2$, -NHC(=O)O-$C_{1-10}$ alkyl, -NHC(=O)$C_{1-10}$ alkyl, -COOH, -C(=O)O-$C_{1-10}$ alkyl, -C(=O)$C_{1-10}$ alkyl, -C(O)H, -S(=O)-$C_{1-10}$ alkyl, -S(=O)$_2$-$C_{1-10}$ alkyl, -S(=O)-aryl, -S(=O)$_2$-aryl, $C_{3-10}$ cycloalkyl optionally substituted with 1, 2 or 3 R8 groups, heterocyclyl optionally substituted with 1, 2 or 3 R8 groups;

R7a is selected from H, trifluoromethyl, $C_1$-$C_{20}$ alkyl, $C_2$-$C_{20}$ alkenyl, $C_2$-$C_{20}$ alkynyl, $C_{3-10}$ cycloalkyl, $C_{3-10}$ cycloalkylalkyl, aryl, arylalkyl, heteroaryl, heteroarylalkyl, heterocyclyl, heterocycloalkyl, wherein said alkyl, alkenyl, alkynyl, cycloalkyl, aryl, heteroaryl and heterocyclyl groups are optionally substituted with one to three substituents independently selected from hydroxy, amino, alkyl or cycloalkyl;

R8 is independently selected from $C_{1-10}$ alkyl, $C_{2-10}$ alkenyl, $C_{2-10}$ alkynyl, phenyl, halogen, haloalkyl, alkoxy, thioalkoxy, amino, alkylamino, dialkylamino, carboxy, $C_{1-4}$ alkyl-OC(=O)-, $C_{1-4}$ alkyl-C(=O)-, aryl-OC(=O)-, $C_{1-4}$ alkyl-OC(=O)NH-, aryl-OC(=O)NH-, $C_{1-4}$ alkyl-C(=O)NH-, $C_{1-4}$ alkyl-C(=O)O-, ($C_{1-4}$ alkyl-O)$_r$- $C_{1-4}$ alkyl, HO-($C_{1-4}$ alkyl-O)$_r$- $C_{1-4}$ alkyl-, -OH, -SH, -CN, $-N_3$, -CNO, -CNS, $C_{1-4}$ alkyl-S(=O)-, $C_{1-4}$ alkyl-S(=O)$_2$-, $H_2NS(=O)$- and $H_2NS(=O)_2$, wherein r is 1 or 2.

Preferred R1 groups are hydrogen, methyl, ethyl, tert-butyl, 2-hydroxyethyl, cyclopropylmethyl, 2-propenyl, carboxymethyl, (R)-2,3-dihydroxypropyl.

Preferred R2 groups are hydrogen, methyl and ethyl.

Preferred R2, R3 and R4 groups are hydrogen, methyl, fluorine, chlorine, bromine, iodine.

Preferred A heterocycles containing from one to three nitrogen, oxygen, sulphur atoms are: pyrrole, furan, thiophene, pyrazole, thiazole, oxazole, imidazole, isothiazole, isoxazole, 1,2,3-triazole, 1,2,4-triazole, 1,2,4-oxadiazole, 1,3,4-oxadiazole, 1,2,5-oxadiazole, 1,2,5-thiadiazole, 1,3,4-thiadiazole, tetrazole, pyridine, pyrimidine, pyridazine, pyrazine, 1,2,4-triazine, 1,2,3-triazine, 1,2,4-triazine, 1,3,5-triazine; even more preferred are pyrrole, furan, thiophene and pyridine.

Preferred R7 groups are hydrogen, methyl, trifluoromethyl, fluorine, chlorine, bromine, iodine.

Among X groups, the NH group is preferred when A is a pyridine ring and the $-CH_2$- and -CO- groups are preferred when A is a thiophene ring.

Particularly preferred are compounds of formula (I) wherein X is NH and A is a pyridine ring, in particular the compounds disclosed in examples 1-71, 73-145, 147-174 and 176-178. Preferred are also the compounds of examples 72, 146 and 175, wherein A is a pyrazine ring, and also the compounds of examples 179-181, wherein A is a thiophene ring.

The compounds of the invention can be prepared from commercially available starting materials using synthetic

methods well known to those skilled in the art.

A useful synthetic procedure for the preparation of the compounds of the invention wherein X is NH comprises the reaction of a heterocyclic amine of formula II

$$A\text{-}NH_2 \qquad (II)$$

wherein A is as defined above
with a benzoic acid of formula III

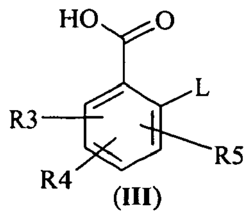

**(III)**

wherein R3, R4 and R5 are as defined above and L is a leaving group, such as halogen or an activated hydroxy group, for example diethylphosphate, trimethylsilyloxy, p-nitrophenoxy, p-toluenesulphonyloxy or methanesulphonyloxy to give a heteroarylamino-benzoic acid of formula IV

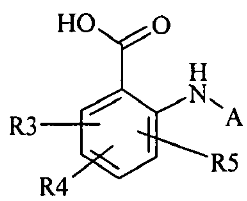

**(IV)**

wherein A, R3, R4 and R5 are as defined above,
followed by treatment of the heteroarylamino-benzoic acid IV with a hydroxylamino derivative V

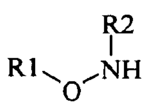

**(V)**

to give the compounds of formula I wherein X is NH.

The reaction of the heterocyclic amine II with benzoic acid III is carried out mixing benzoic acid III with equimolar amounts or with a slight excess of the heterocyclic amine III in an organic solvent such as tetrahydrofuran or toluene, in the presence of a base such as lithium diisopropylamide, n-butyl lithium, sodium hydride or sodium amide. The reaction is usually carried out at a temperature comprised between about -78°C and about 80°C and is usually complete in a time comprised between four hours and four days.

Heteroarylamino benzoic acid IV is then reacted with hydroxylamine V in the presence of a condensing agent such as, 2-ethoxy-1-ethoxycarbonyl-1,2-dihydroquinoline (EEDQ), 1,3-dicyclohexylcarbodiimide (DCC), bromo-tris(pyrrolidino)phosphonium hexafluorophosphate (PyBrOP), (benzotriazolyloxy)trispyrrolidinophosphonium hexafluorophosphate (PyBOP), HOBt, EDCI, diphenylphosphinic chloride. The compound of formula IV and the compound of formula V are usually mixed in equimolar amounts, optionally in the presence of a base such as triethylamine or diisopropylamine, in an organic solvent such as dichloromethane, tetrahydrofuran, chloroform, xylene, followed by addition of an equimolar amount of the condensing agent. The reaction is usually complete in a time period ranging from ten minutes to two hours.

Alternatively, the heteroarylamino benzoic acid IV can be converted, with conventional methods, into the corresponding acyl chloride, mixed anhydride or activated esters such as, for example, the N-hydroxysuccinimido ester, p-nitrophenyl ester, pentafluorophenyl ester or a thioester, followed by addition of the compound of formula V.

An alternative process for the preparation of the compounds of formula I in which X is NH comprises the reaction of a compound of formula III with a compound of formula V, to give a hydroxamic derivative VI

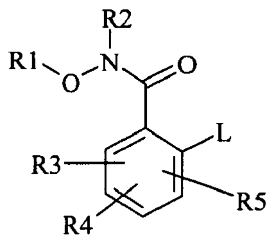

(VI)

in which, R1, R2, R3, R4, R5 and L are as defined above, followed by reaction of the compound of formula VI with a heterocyclic amine of formula II to give compounds of formula I in which X is NH.

The reaction of compounds of formula III with compounds of formula V can be carried out according to the methods described above for the reaction of the compounds of formula IV with the compounds of formula V. The reaction of the compounds of formula VI with the compounds of formula II can be carried out according to the methods described above for the reaction between the compounds of formula II with the compounds of formula IV.

In some cases, the compound of formula V used in the condensation reaction with the compounds of formula IV or III may contain a conventional protective group. For example, protective groups than can be advantageously used for the protection of a hydroxy group are vinyl ethers, silyl groups, acetals, acetonides and carbamates. In this case the protective group can be removed using conventional methods known in organic chemistry.

The compounds of formula II and III are known and are commercially available or can be prepared with known methods. The hydroxylamino derivatives of formula V are known or can be prepared with known methods, for example according to the procedures disclosed in WO 02/06213.

Thanks to their ability to selectively inhibit protein-kinases MEK1 and MEK2, the compounds of the invention are useful for the treatment of tumour pathologies and other proliferative disorders. The compounds of the invention have been evaluated in biological tests conventionally used for evaluating the inhibition of protein kinases and measuring the cellular effects due to this inhibition.

For example, the ability of the compounds of the invention to inhibit MEK can be determined with the procedure disclosed in WO 2005/051906 or using a commercially available kit, for example the MEK Activity Assay Kit (Sigma, product code CS0490). In these tests, the compounds of the invention show an $IC_{so}$ lower than 50 $\mu$M for the inhibition of MEK.

The antiproliferative effects of the compounds of the invention on human tumours lines from different cell histotypes (for example, colon, melanoma, lung, prostate, ovary, breast, leukemia, lymphoma, myeloma) can be evaluated with a number of methods well known to those skilled in the art, for example incubating the cells with the compounds for 144 hours and then determining their cytotoxicity with the MTT assay (Mosman, T., J. Immunolog. Methods, (1983), 65, 66; Green, L.M., J. Immunol. Methods, (1984), 70. 257-268). In this test the compounds of the invention showed $IC_{50}$ values (compound concentration necessary to induce 50% cell death) lower than 50 $\mu$M.

As an alternative, tumour cells are incubated with the compounds for 96 hours and after this time cell proliferation is determined using the ATP lite cell proliferation kit (Packard).

The compounds of the invention have $IC_{50}$ values (compound concentration necessary to induce 50% cell death) lower than 50 $\mu$M.

A further object of the invention are pharmaceutical compositions for the treatment of hyperproliferative pathologies in a mammal, the compositions comprising a therapeutically effective amount of a compound of the invention, or a pharmaceutically acceptable salt, pro-drug or hydrate thereof, and one or more pharmaceutically acceptable excipients. These pharmaceutical compositions are particularly useful for the treatment of brain, lung, squamous cells, bladder, stomach, pancreas, breast, head, neck, kidney, ovary, prostate, colon-rectum, esophagus, testicles, thyroid tumours or for the treatment of gynaecological tumours. According to a further aspect, the compositions can contain, further to a compound of formula I, another chemotherapeutic agent in such an amount to effectively inhibit, together with the compound of formula I, anomalous cell proliferation. Chemotherapeutic agents that can be effectively combined with the compounds of the invention are for example mitosis inhibitors, alkylating agents, antimetabolites, intercalating agents, antibiotics, growth factors inhibitors, cell cycle inhibitors, topoisomerase inhibitors, anti-hormonal and angiogenesis inhibitors. The compositions of the invention can be prepared with excipients and methods known to those skilled in the art, for example those described in Remington's Pharmaceutical Sciences Handbook, Mack Pub., N.Y., U.S.A..

A further object of the invention is a method for the treatment of hyperproliferative disorders in a mammal, the method comprising administering to said mammal a therapeutically effective amount of a compound of the invention, or a pharmaceutically acceptable salt thereof, a pro-drug or a hydrate thereof. In an aspect, the method relates to the treatment of tumour pathologies of the brain, lung, squamous cells, bladder, stomach, pancreas, breast, head, neck, kidney, ovary, prostate, colon-rectum, esophagus, testicles, and thyroid, or for the treatment of gynaecological tumours.
The invention is illustrated in greater detail in the following experimental section.

## EXPERIMENTAL SECTION

## PREPARATION OF ACIDS OF FORMULA IV

### Preparation 1: 3,4-Difluoro-2-(3,5-dichloro-2-pyridylamino)benzoic acid

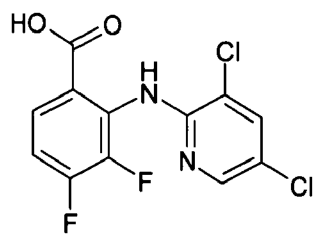

Under nitrogen atmosphere, a solution of 3,5-dichloro-2-pyridylamine (1.63 g, 10 mmol; Aldrich, code 135925) and 2,3,4-trifluorobenzoic acid (1.76 g, 10 mmol; Aldrich, code 333824) in anhydrous THF (15 ml) cooled to -78˚C is added drop by drop with a solution of 1.06 M of LiHMDS (lithium hexamethyldisilazane) in THF (28.4 ml, 30 mmol). At the end of the addition the reaction mixture is allowed to warm to room temperature and left under stirring for 24 hours. After evaporation of the solvent under reduced pressure, the residue is divided and taken up in HC1 1M (50 ml) and ethyl acetate (2 x 50 ml). The organic phases are pooled and washed in sequence with water (50 ml), with a saturated NaCl solution (50 ml) and anhydrified over $Na_2SO_4$. After evaporation of the solvent the residue is purified by chromatography on silica gel eluting with ethyl acetate and with a 9/1 ethyl acetate/methanol mixture. The product-containing fractions are pooled and the solvent is evaporated off to give 3,4-difluoro-2-(3,5-dichloro-2-pyridylamino)benzoic acid.

### Preparations 2 - 67

The compounds of table 1 are prepared following the procedure of preparation 1 and using suitable benzoic acids and heterocyclic amines as starting products.

| Preparation | Structure |
|---|---|
| 2. | 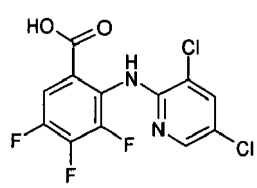 |
| 3. | 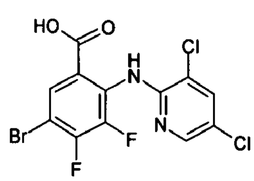 |

(continued)

| Preparation | Structure |
|---|---|
| 4. | 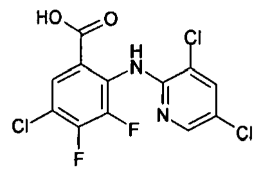 |
| 5. | 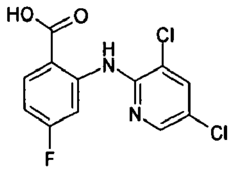 |
| 6. | 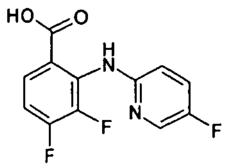 |
| 7. | 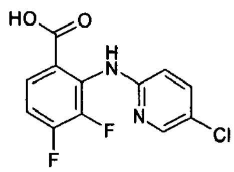 |
| 8. | 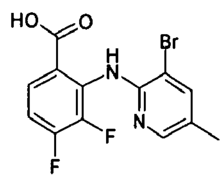 |
| 9. | 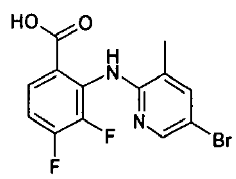 |
| 10. | 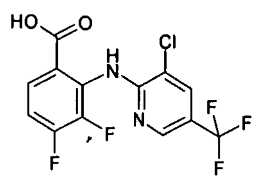 |
| 11. | 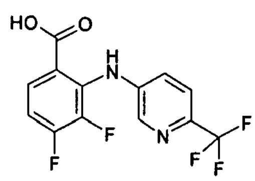 |

(continued)

| Preparation | Structure |
|---|---|
| 12. | 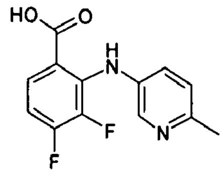 |
| 13. | 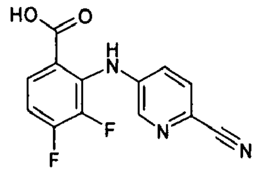 |
| 14. | 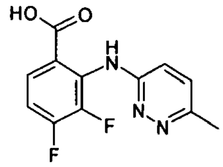 |
| 15. | 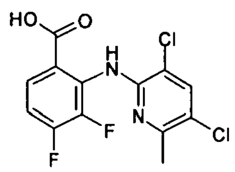 |
| 16. | 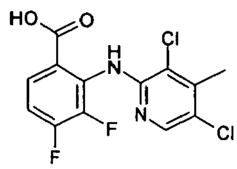 |
| 17. | 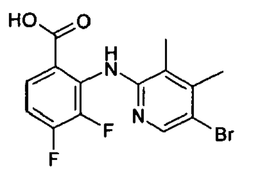 |
| 18. | 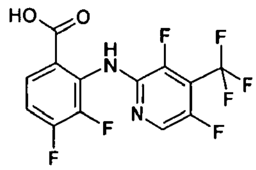 |
| 19. | 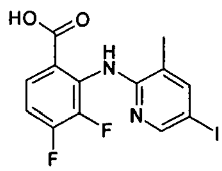 |

(continued)

| Preparation | Structure |
|---|---|
| 20. | 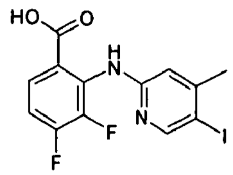 |
| 21. | 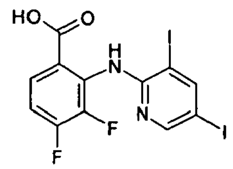 |
| 22. | 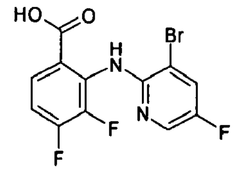 |
| 23. | 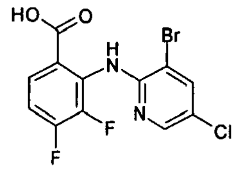 |
| 24. | 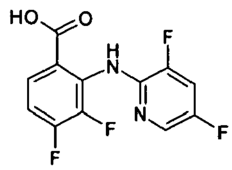 |
| 25. | 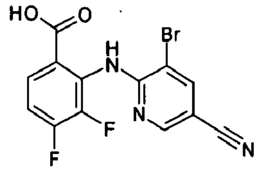 |
| 26. | 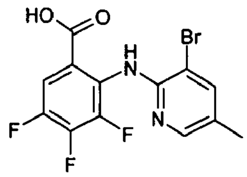 |
| 27. | 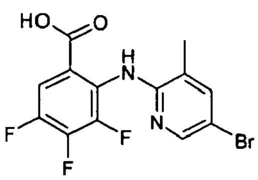 |

(continued)

| Preparation | Structure |
|---|---|
| 28. | 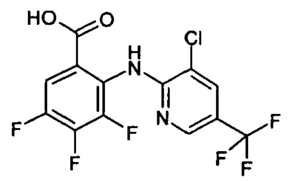 |
| 29. | 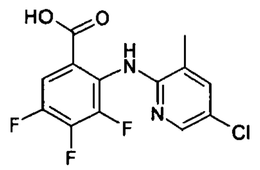 |
| 30. | 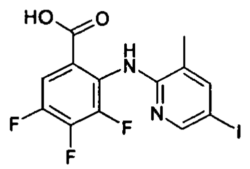 |
| 31. | 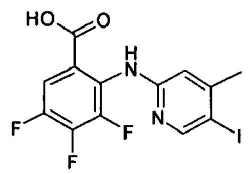 |
| 32. | 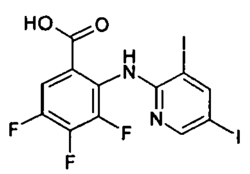 |
| 33. | 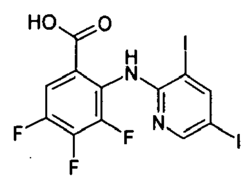 |
| 34. | 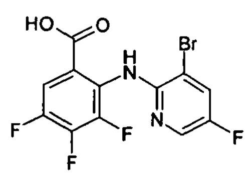 |
| 35. | 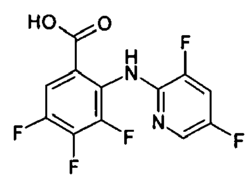 |

(continued)

| Preparation | Structure |
|---|---|
| 36. | 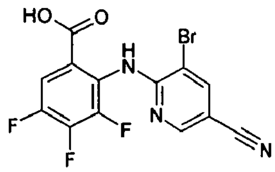 |
| 37. | 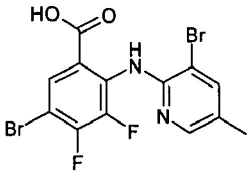 |
| 38. | 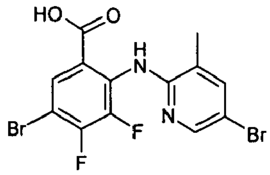 |
| 39. | 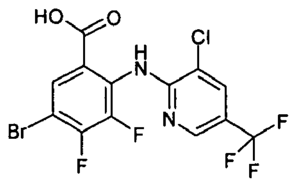 |
| 40. | 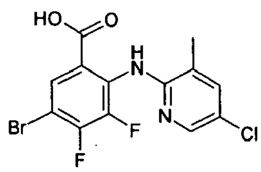 |
| 41. | 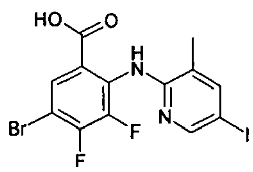 |
| 42. | 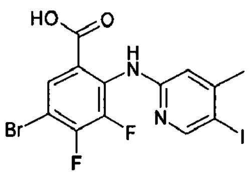 |
| 43. | 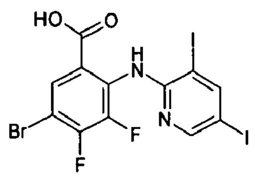 |

(continued)

| Preparation | Structure |
|---|---|
| 44. | 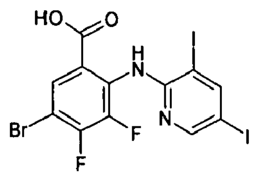 |
| 45. | 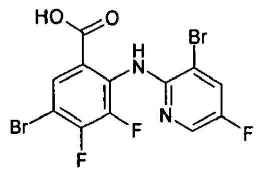 |
| 46. | 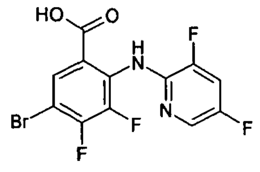 |
| 47. | 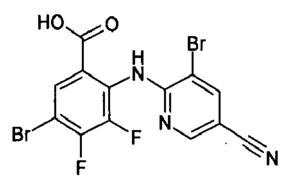 |
| 48. | 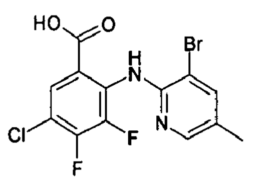 |
| 49. | 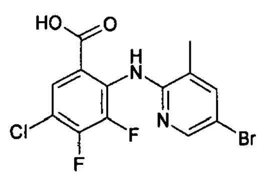 |
| 50. | 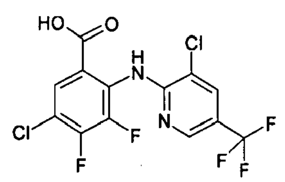 |
| 51. | 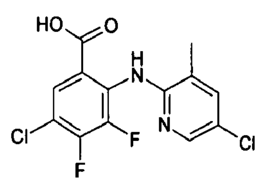 |

(continued)

| Preparation | Structure |
|---|---|
| 52. | 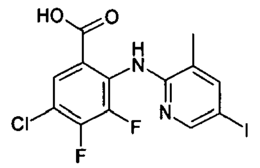 |
| 53. | 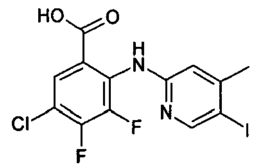 |
| 54. | 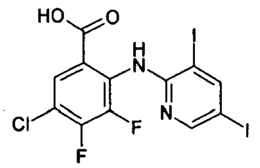 |
| 55. | 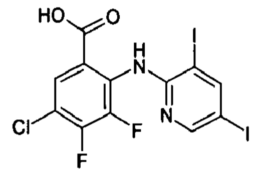 |
| 56. | 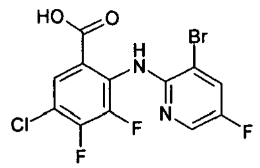 |
| 57. | 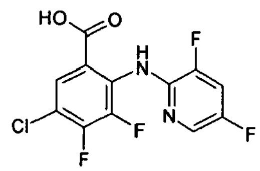 |
| 58. | 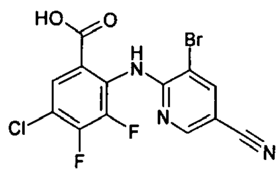 |
| 59. | 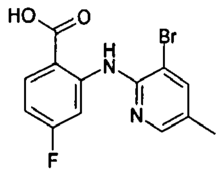 |
| 60. | 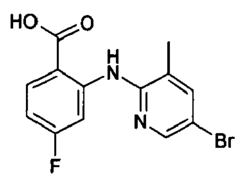 |

(continued)

| Preparation | Structure |
|---|---|
| 61. | 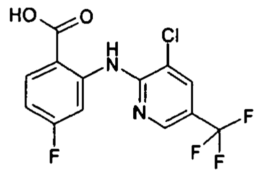 |
| 62. | 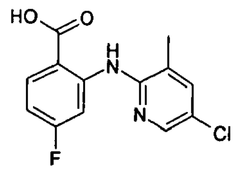 |
| 63. | 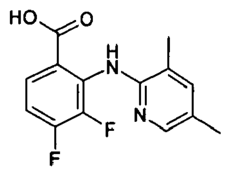 |
| 64. | 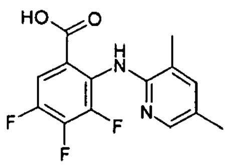 |
| 65. | 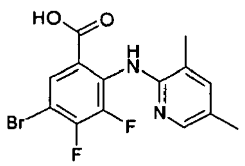 |
| 66. | 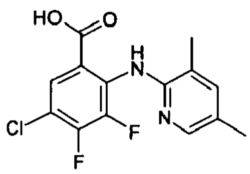 |
| 67. | 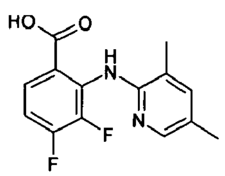 |

**<u>Example 1</u>: 2-(3,5-Dichloro-2-pyridylamino)-3,4-difluoro-N-methoxy-N-methylbenzamide**

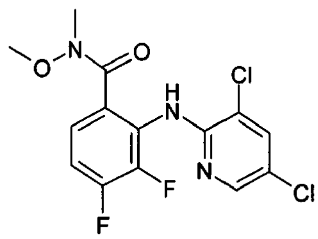

3,4-Difluoro-2-(3,5-dichloro-2-pyridylamino)benzoic acid from preparation 1 (0.319 g, 1.0 mmol) is dissolved in dichloromethane (5 ml) and N-methylmorpholine (0.6 ml) and the resulting solution is added with N,O-dimethylhydroxylamine hydrochloride (0.127 g, and 1.30 mmol) and (benzotriazolyloxy)trispyrrolidino phosphonium hexafluorophosphate (PyBOP; 0.52 g, 1.0 mmol). The reaction mixture is stirred for 4 hours at room temperature. A further portion of PyBOP (0.17 g; 0.33 mmol) is added and stirring is continued at room temperature for 24 hours. After evaporation of the reaction solvent under reduced pressure, the residue is dissolved in ethyl acetate (25 ml) and the organic solution is washed in sequence with water (25 ml) and with a saturated solution of NaCl (3 x 25 ml). The organic phase is anhydrified over $Na_2SO_4$, the solvent is removed under reduced pressure and the residue is purified by chromatography on silica gel to afford the product.

**Example 2: N-(2-Hydroxyethoxy)-2-(3,5-dichloro-2-pyridylamino)-3,4-difluorobenzamide**

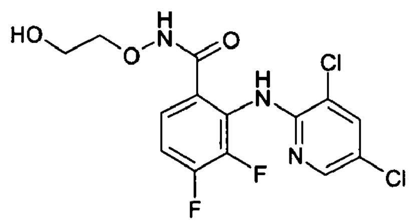

**Step 1**

A solution of 3,4-difluoro-2-(3,5-dichloro-2-pyridylamino)benzoic acid of preparation 1 (0.319 g, 1.0 mmol) and HOBt (0.203 g 1.5 mmol) in dichloromethane (5 ml) is added with EDCI (0.233 g, 1.5 mmol), then left under stirring for 2 hours at room temperature and added with O-(2-vinyloxyethyl)hydroxylamine (0.113 g. 1.1 mmol; obtained according to preparation 47 of WO02/06213) and triethylamine (0.17 ml, 1.2 mmol). The reaction mixture is stirred for two hours. The reaction solvent is evaporated off under reduced pressure, the residue is dissolved in ethyl acetate (25 ml) and the organic solution is washed in sequence with an ammonium chloride saturated solution (25 ml), a NaCl saturated solution (25 ml), a $NaHCO_3$ saturated solution (25 ml) and finally with a NaCl saturated solution (25 ml). The organic phase is anhydrified over $Na_2SO_4$, the solvent is removed under reduced pressure and the obtained residue of N-(2-vinyloxyethoxy)-2-(3,5-dichloro-2-pyridylamino)-3,4-difluorobenzamide is used for the following step without further purification.

**Step 2**

A solution of the product from step 1 (0.404 g; 1.0 mmol) in THF/EtOH 1/1 is added at room temperature with 1N HCl (2 mL; 2.0 mmol). The reaction mixture is stirred at room temperature for about one hour, then neutralised by addition of a saturated $NaHCO_3$ solution and diluted with ethyl acetate (25 ml). The organic solution is washed with a NaCl saturated solution (25 ml), anhydrified over $Na_2SO_4$, the solvent is evaporated off under reduced pressure and the residue is purified by column chromatography to give N-(2-hydroxyethoxy)-2-(3,5-dichloro-2-pyridylamino)-3,4-difluorobenzamide.

**Example 3: N-[(R)-2,3-Dihydroxypropoxy]-2-(3,5-dichloro-2-pyridylamino)-3,4-difluorobenzamide**

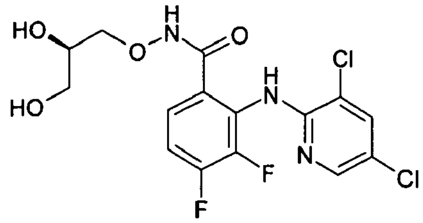

**Step 1**

A solution of 3,4-difluoro-2-(3,5-dichloro-2-pyridylamino)benzoic acid of preparation 1 (0.319 g, 1.0 mmol) in anhydrous THF (5 ml) is added with (R)-O-(2,2-dimethyl-1,3-dioxolan-4-yl-methyl)hydroxylamine (0.147 g; 0.1 mmol; prepared according to preparations 70 and 71 of WO 02/06213), followed by N-methylmorpholine (0.28 ml, 2.5 mmol). The solution is cooled to 0°C and added with chlorodiphenylphosphine oxide (0.23 ml, 1.2 mmol). The reaction mixture is allowed to warm to room temperature and left under stirring for 24 hours. After removal of the solvent under reduced pressure, the residue is partitioned between water (25 ml) and ethyl acetate (25 ml). The organic phase is separated and washed twice with a 1/1 NaCl/$NaHCO_3$ saturated solution. After anhydrification on $Na_2SO_4$, the solvent is removed under reduced pressure and the residue is purified by column chromatography to give N-[(R)-2,2-dimethyl-1,3-dioxolan-4-yl-methoxy]-2-(3,5-dichloro-2-pyridylamino)-3,4-difluorobenzamide.

**Step 2**

A mixture of product of step 1 (0.224 mg; 0.5 mmol), p-toluenesulphonic acid monohydrate (0.048 g, 0.25 mmol), methanol (2.5 ml) and water (0.25 ml) is stirred at room temperature for 24 hours. The reaction mixture is concentrated under reduced pressure and the residue is extracted with ethyl acetate (2 x 10 ml). The organic phases are pooled and washed with a $NaHCO_3$ saturated solution and dried over $Na_2SO_4$. After evaporation of the solvent the residue is purified by column chromatography to give N-[(R)-2,3-dihydroxypropoxy]-2-(3,5-dichloro-2-pyridylamino)-3,4-difluorobenzamide.

**Examples 4-178**

The compounds reported below in table 2 are prepared following the procedures of Examples 1-3 and using the heteroarylaminobenzoic acids of preparations 1-67 and suitable hydroxylamines as starting products.

**Table 2**

| Example | Structure |
|---|---|
| 4. | 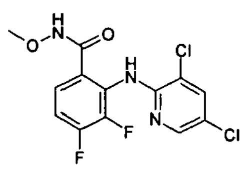 |
| 5. | 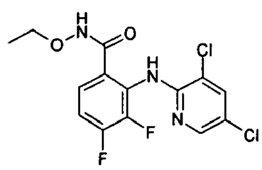 |

(continued)

| Example | Structure |
|---|---|
| 6. | 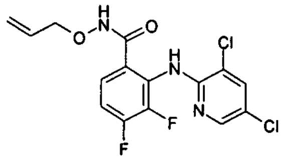 |
| 7. | 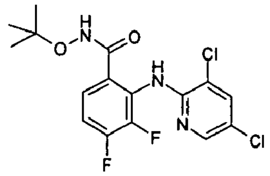 |
| 8. | 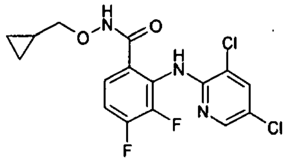 |
| 9. | 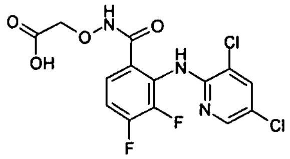 |
| 10. | 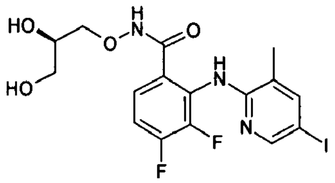 |
| 11. | 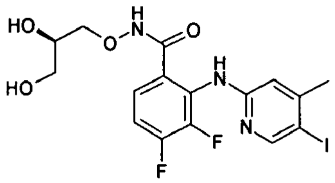 |
| 12. | 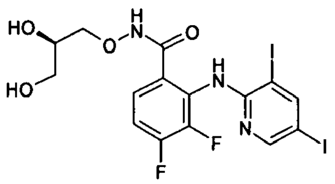 |
| 13. | 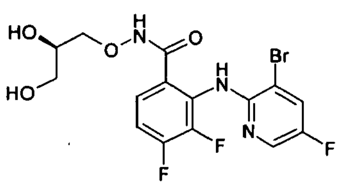 |

(continued)

| Example | Structure |
|---|---|
| 14. | 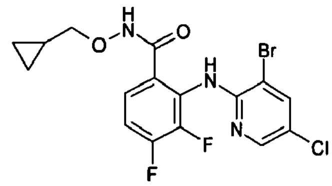 |
| 15. | 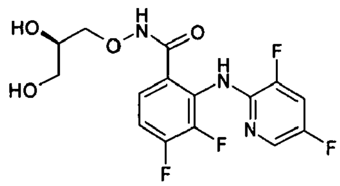 |
| 16. | 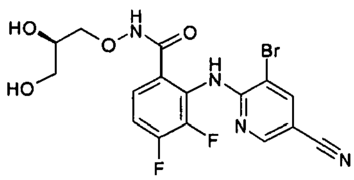 |
| 17. | 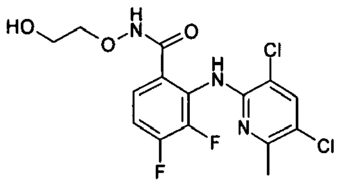 |
| 18. | 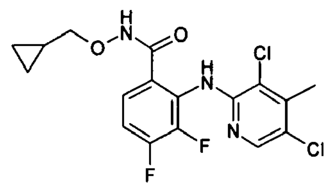 |
| 19. | 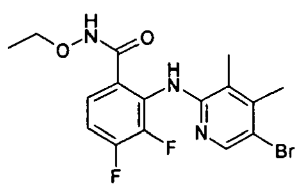 |
| 20. | 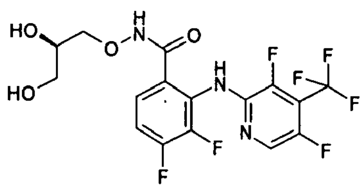 |
| 21. | 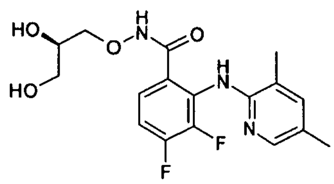 |

(continued)

| Example | Structure |
|---|---|
| 22. | 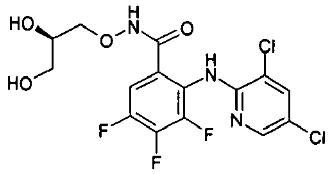 |
| 23. | 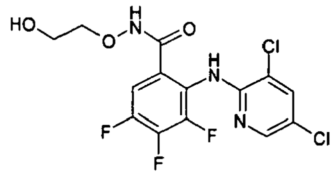 |
| 24. | 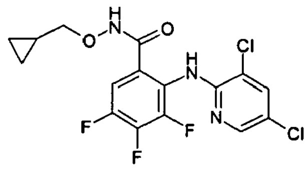 |
| 25. | 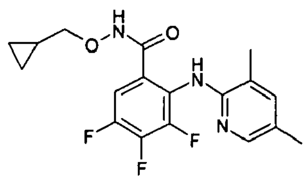 |
| 26. | 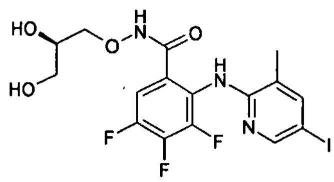 |
| 27. | 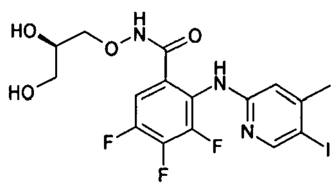 |
| 28. | 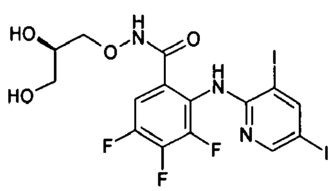 |
| 29. | 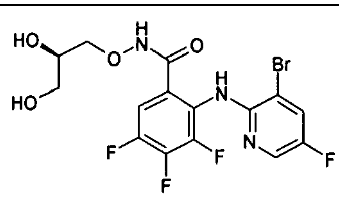 |

(continued)

| Example | Structure |
|---|---|
| 30. | 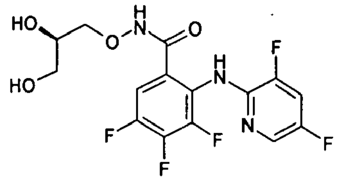 |
| 31. | 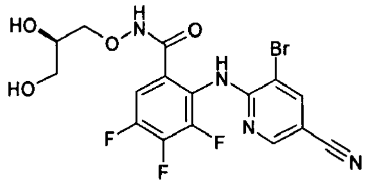 |
| 32. | 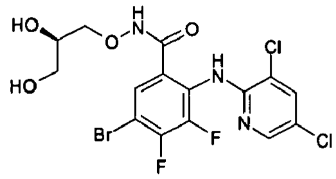 |
| 33. | 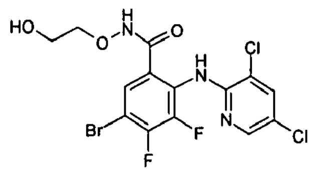 |
| 34. | 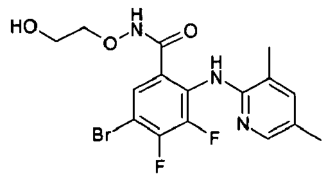 |
| 35. | 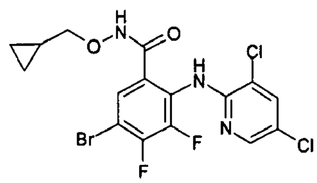 |
| 36. | 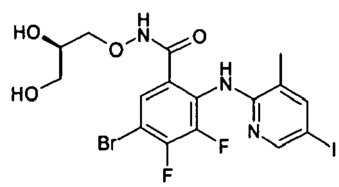 |
| 37. | 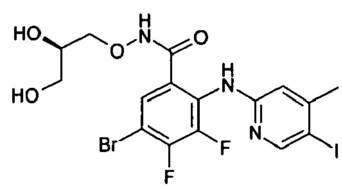 |

(continued)

| Example | Structure |
|---|---|
| 38. | 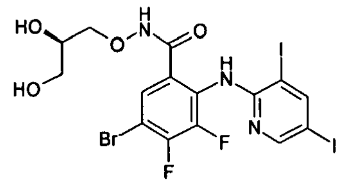 |
| 39. | 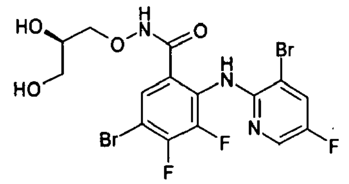 |
| 40. | 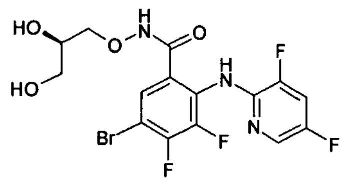 |
| 41. | 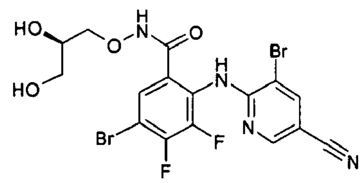 |
| 42. | 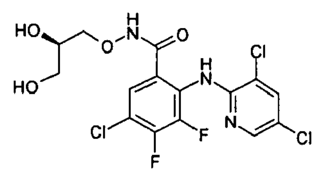 |
| 43. | 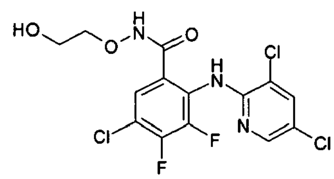 |
| 44. | 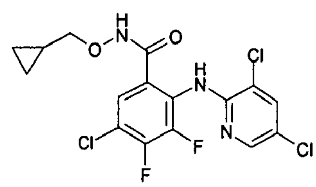 |
| 45. | 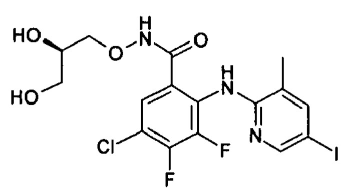 |

(continued)

| Example | Structure |
|---|---|
| 46. | 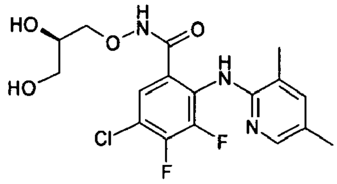 |
| 47. | 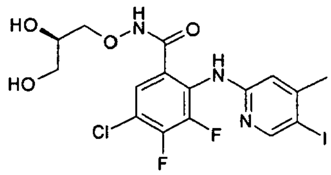 |
| 48. | 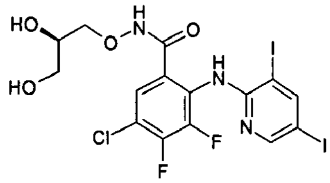 |
| 49. | 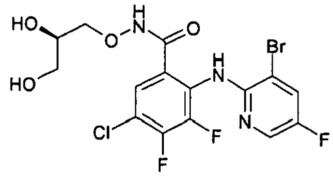 |
| 50. | 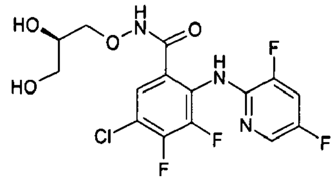 |
| 51. | 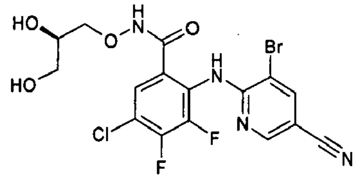 |
| 52. | 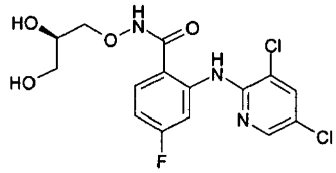 |
| 53. | 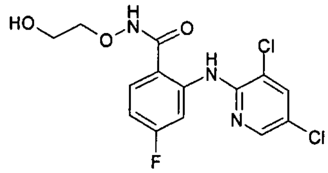 |

(continued)

| Example | Structure |
|---|---|
| 54. | 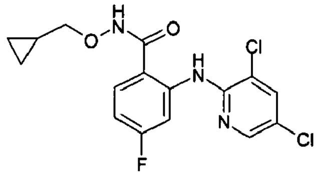 |
| 55. | 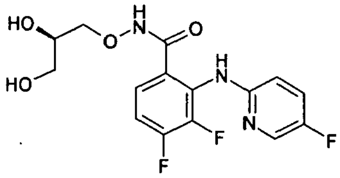 |
| 56. | 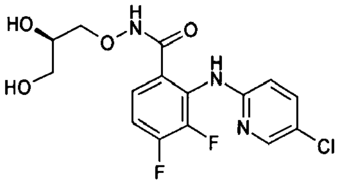 |
| 57. | 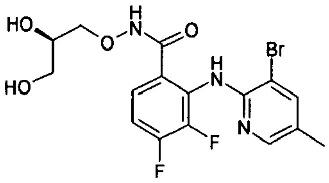 |
| 58. | 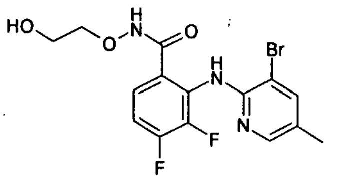 |
| 59. | 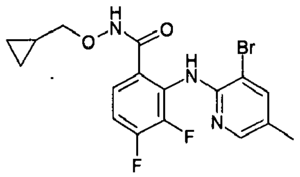 |
| 60. | 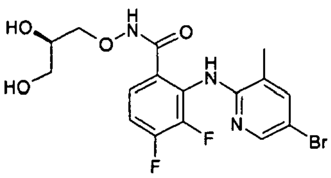 |
| 61. | 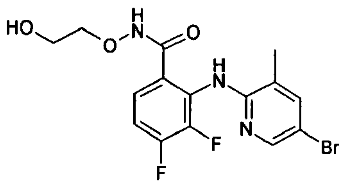 |

(continued)

| Example | Structure |
|---|---|
| 62. | 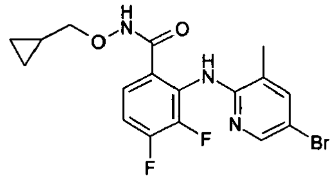 |
| 63. | 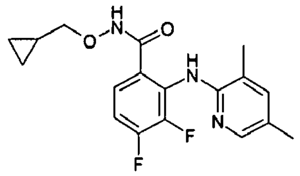 |
| 64. | 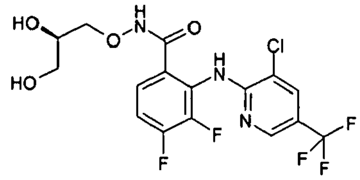 |
| 65. | 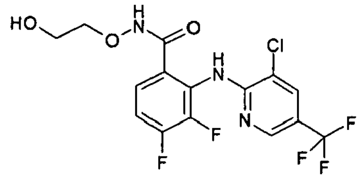 |
| 66. | 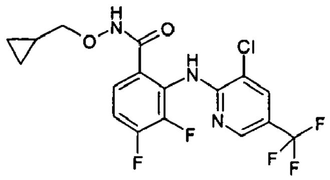 |
| 67. | 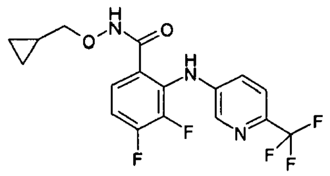 |
| 68. | 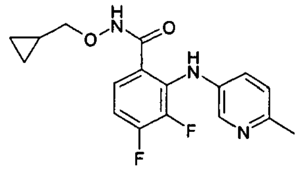 |
| 69. | 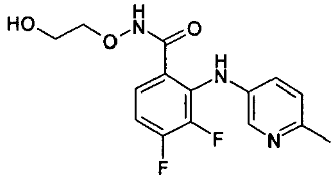 |

(continued)

| Example | Structure |
|---|---|
| 70. | 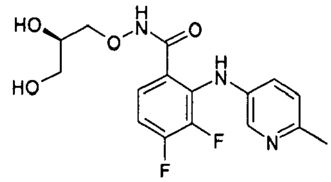 |
| 71. | 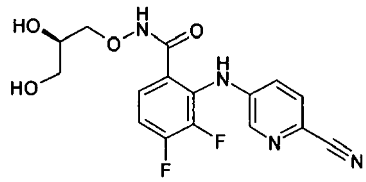 |
| 72. | 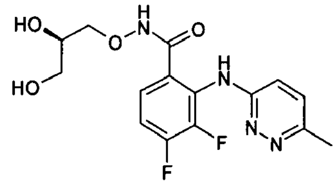 |
| 73. | 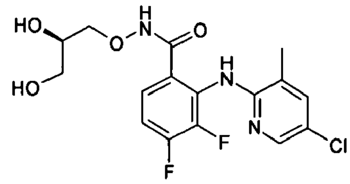 |
| 74. | 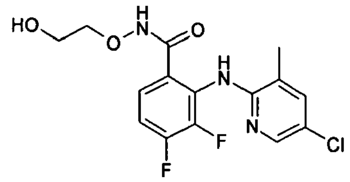 |
| 75. | 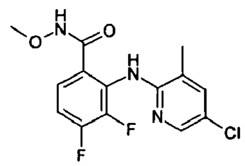 |
| 76. | 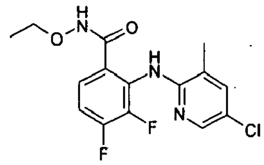 |
| 77. | 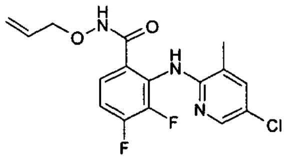 |

(continued)

| Example | Structure |
|---|---|
| 78. | 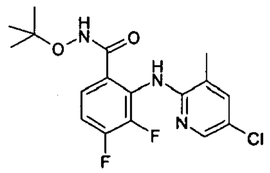 |
| 79. | 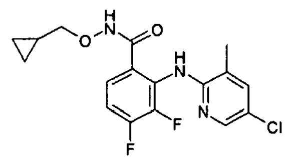 |
| 80. | 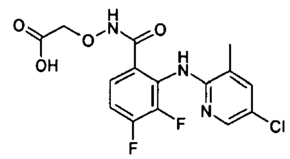 |
| 81. | 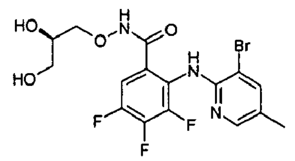 |
| 82. | 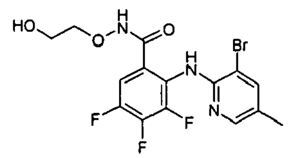 |
| 83. | 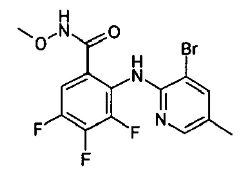 |
| 84. | 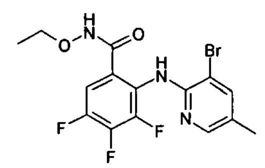 |
| 85. | 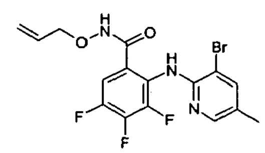 |
| 86. | 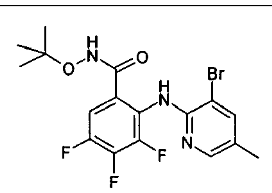 |

(continued)

| Example | Structure |
|---|---|
| 87. | 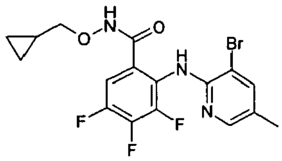 |
| 88. | 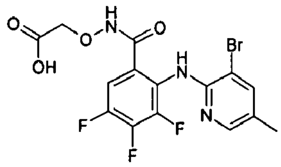 |
| 89. | 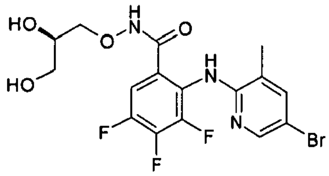 |
| 90. | 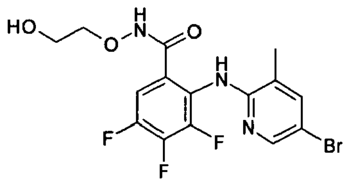 |
| 91. | 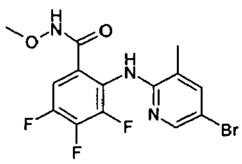 |
| 92. | 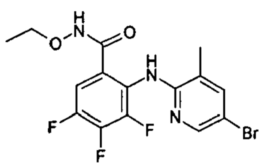 |
| 93. | 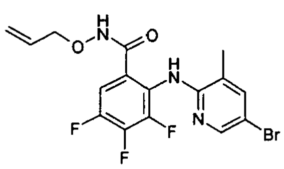 |
| 94. | 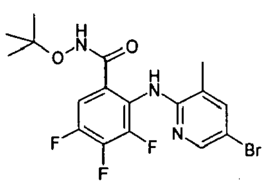 |
| 95. | 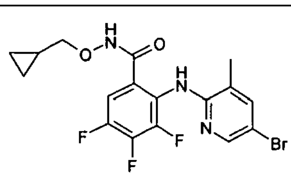 |

(continued)

| Example | Structure |
|---|---|
| 96. | 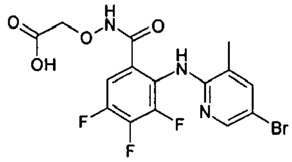 |
| 97. | 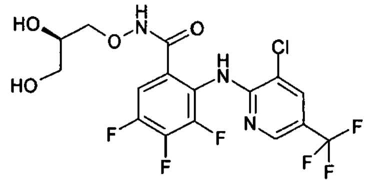 |
| 98. | 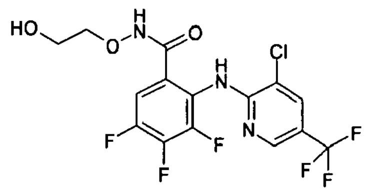 |
| 99. | 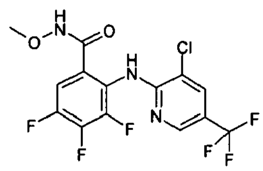 |
| 100. | 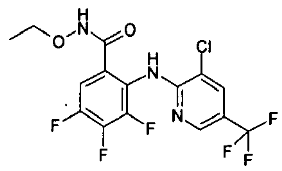 |
| 101. | 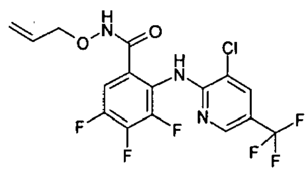 |
| 102. | 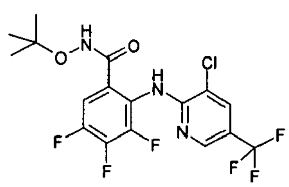 |
| 103. | 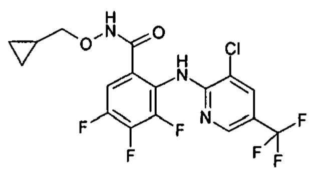 |

(continued)

| Example | Structure |
|---|---|
| 104. | 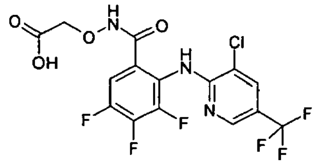 |
| 105. | 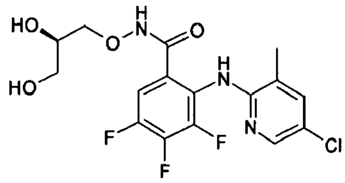 |
| 106. | 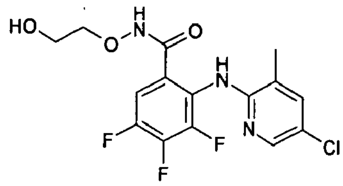 |
| 107. | 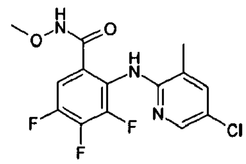 |
| 108. | 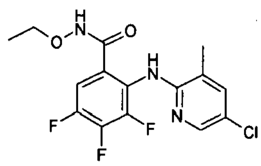 |
| 109. | 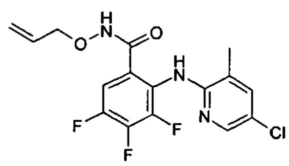 |
| 110. | 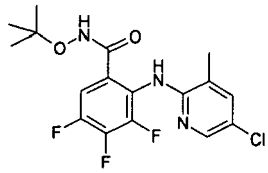 |
| 111. | 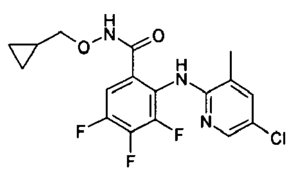 |
| 112. | 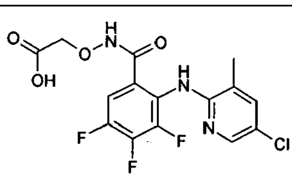 |

(continued)

| Example | Structure |
|---|---|
| 113. | 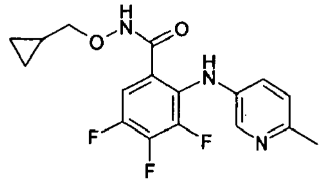 |
| 114. | 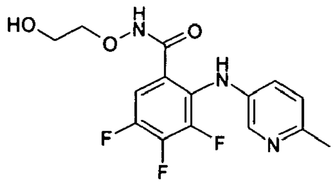 |
| 115. | 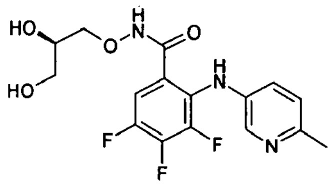 |
| 116. | 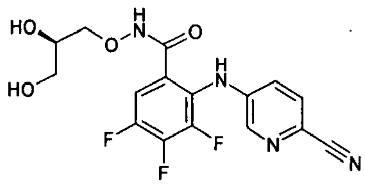 |
| 117. | 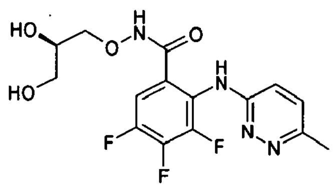 |
| 118. | 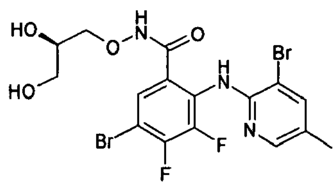 |
| 119. | 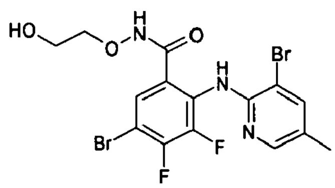 |
| 120. | 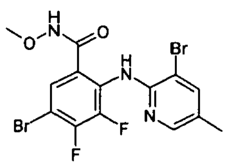 |

(continued)

| Example | Structure |
|---|---|
| 121. | 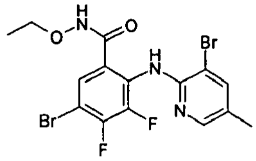 |
| 122. | 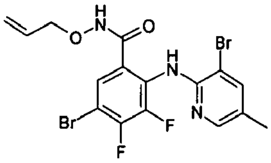 |
| 123. | 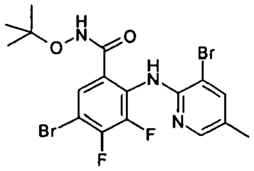 |
| 124. | 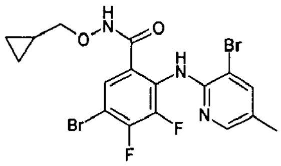 |
| 125. | 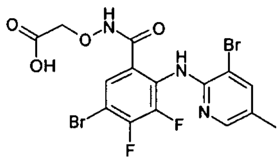 |
| 126. | 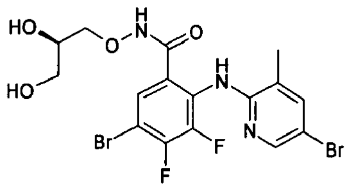 |
| 127. | 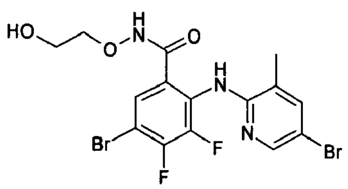 |
| 128. | 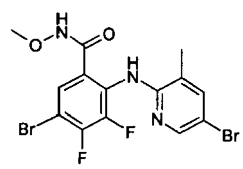 |
| 129. | 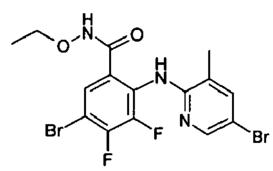 |

(continued)

| Example | Structure |
|---|---|
| 130. | 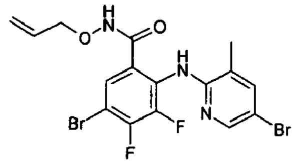 |
| 131. | 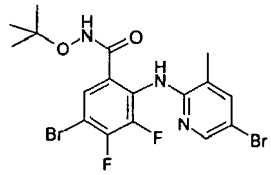 |
| 132. | 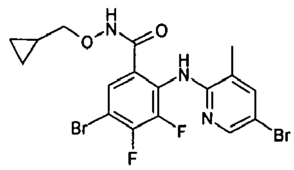 |
| 133. | 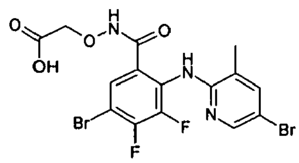 |
| 134. | 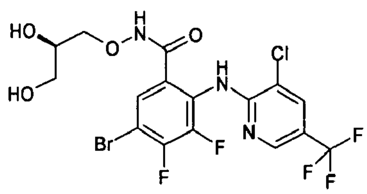 |
| 135. | 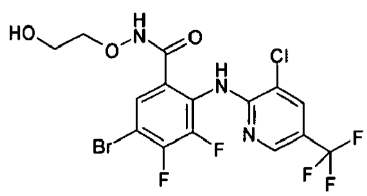 |
| 136. | 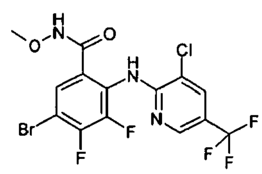 |
| 137. | 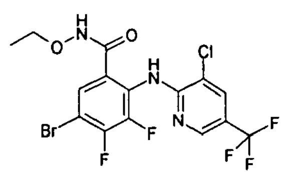 |

(continued)

| Example | Structure |
|---|---|
| 138. | 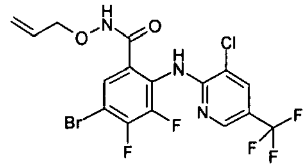 |
| 139. | 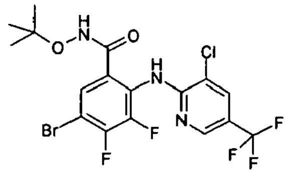 |
| 140. | 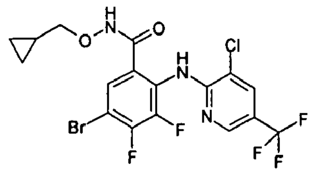 |
| 141. | 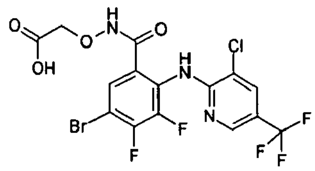 |
| 142. | 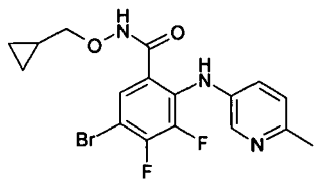 |
| 143. | 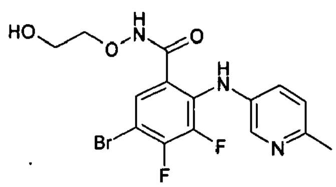 |
| 144. | 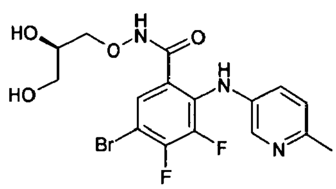 |
| 145. | 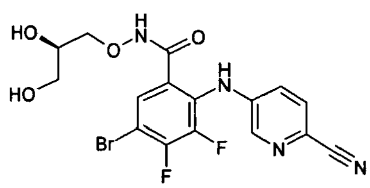 |

(continued)

| Example | Structure |
|---|---|
| 146. | 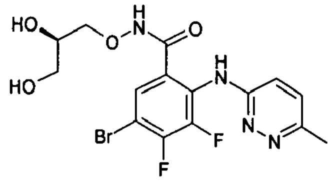 |
| 147. | 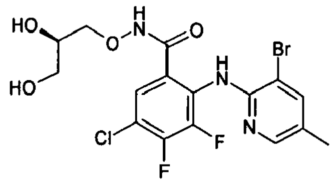 |
| 148. | 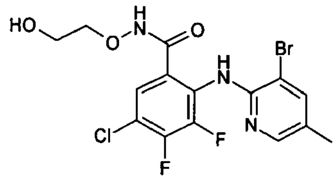 |
| 149. | 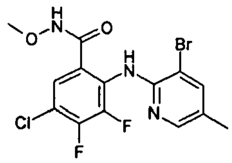 |
| 150. | 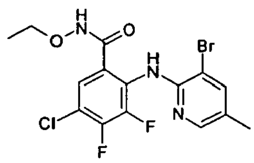 |
| 151. | 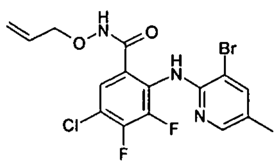 |
| 152. | 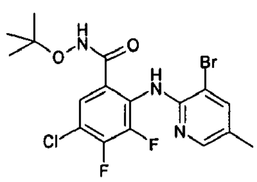 |
| 153. | 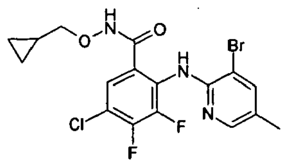 |
| 154. | 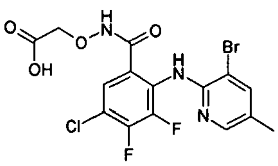 |

(continued)

| Example | Structure |
|---|---|
| 155. | 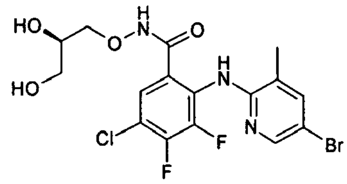 |
| 156. | 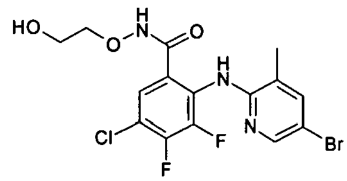 |
| 157. | 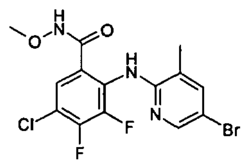 |
| 158. | 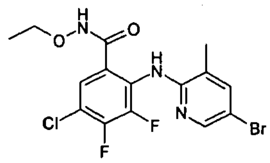 |
| 159. | 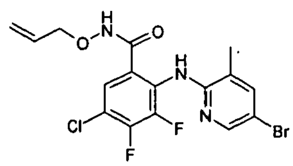 |
| 160. | 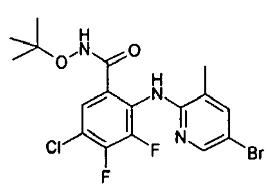 |
| 161. | 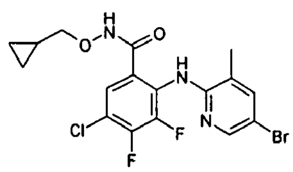 |
| 162. | 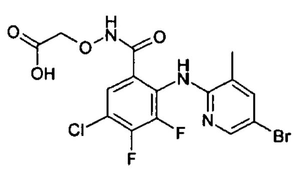 |

(continued)

| Example | Structure |
|---|---|
| 163. | 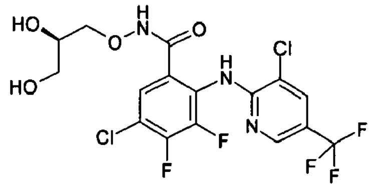 |
| 164. | 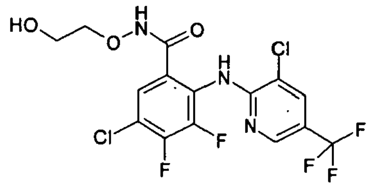 |
| 165. | 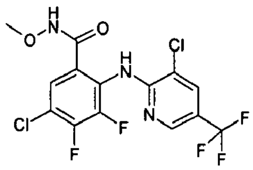 |
| 166. | 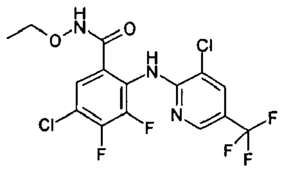 |
| 167. | 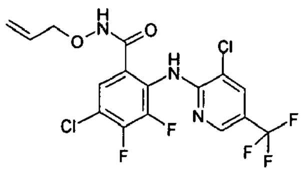 |
| 168. | 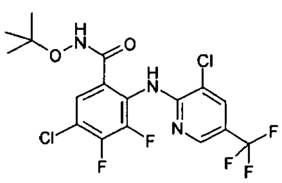 |
| 169. | 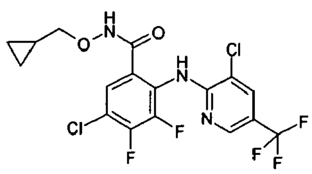 |
| 170. | 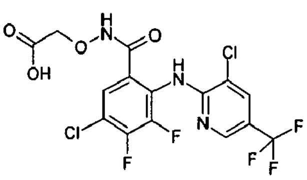 |

(continued)

| Example | Structure |
|---|---|
| 171. | 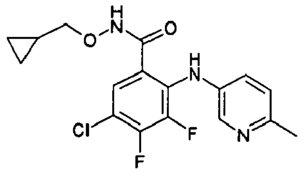 |
| 172. | 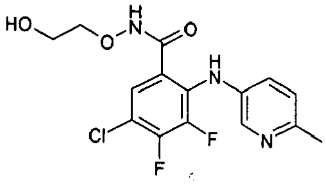 |
| 173. | 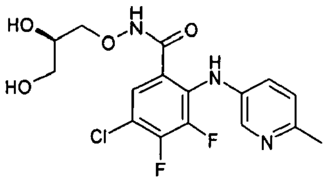 |
| 174. | 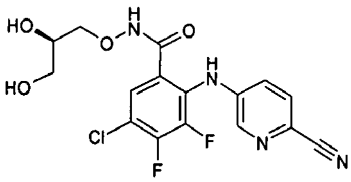 |
| 175. | 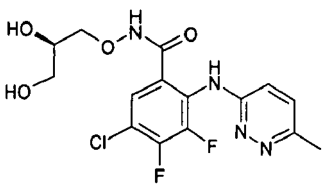 |
| 176. | 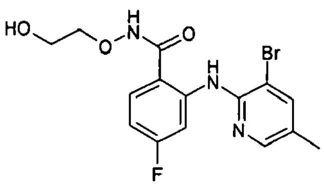 |
| 177. | 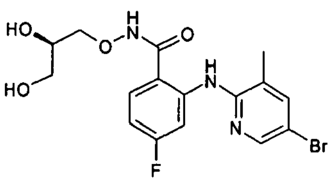 |
| 178. | 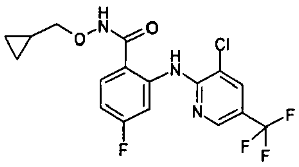 |

**<u>Example 179:</u> N-((R)-2,3-Dihydroxypropoxy)-2-(3,4,5-trichlorothiophene-2-carbonyl)benzamide**

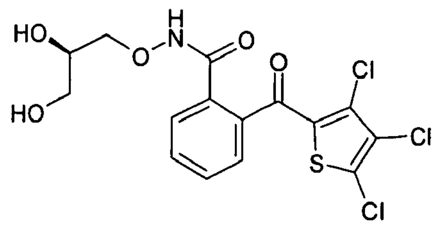

This compound is prepared following the procedure of example 1, using 2-(3,4,5-trichlorothiophene-2-carbonyl) benzoic acid (commercially available from Aurora Fine Chemicals) as starting material.

**Example 180: N-((R)-2,3-Dihydroxypropoxy)-2-(thiophene-2-carbonyl)benzamide**

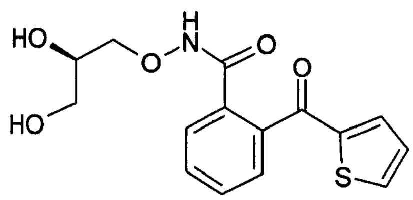

The product is prepared following the procedure of example 1, using 2-(thiophene-2-carbonyl)benzoic acid, which is commercially available, as starting material.

**Example 181: N-((R)-2,3-Dihydroxypropoxy)-2-(thiophene-2-methyl)benzamide**

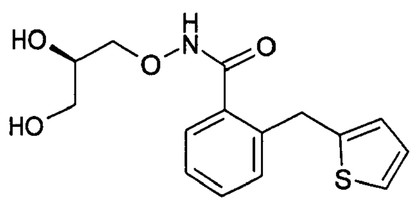

This compound is prepared following the procedure of example 1, using 2-(thiophene-2-methyl)benzoic acid (commercially available from Asinex) as starting material.

**PHARMACOLOGICAL SECTION**

*In vivo* evaluation of antitumour activity

*Materials*

The MEK inhibitors used for *in vivo* antitumour activity studies can be formulated in a suitable medium for endovenous (ev) or oral administration (os). For example, for endovenous administration the compounds can be administered in 0.9% NaCl or in 0.9%NaCl, solutol HS15 and dimethylsulphoxide mixtures, for example in 87:10:3 (v:v:v) ratio respectively.

*Cell lines*

The following murine and human tumour cells from different tissue hystotypes can be used to evaluate the antitumour activity of the compounds of the invention: A375 (human, melanoma), H460 (human, lung), A2780 (human, ovary), PC-3 (human, prostate), LoVo (human, colon), HCT116 (human, colon), BXPC3 (human, pancreas), PANC-1 (human, pancreas), MX-1 (human, breast), MOLT (human, leukemia), multiple myeloma (human, myeloma), YC8 (murine, lymphoma), L1210 (murine, leukemia), 3LL (murine, lung).

*Animal species*

Immunocompetent or immunosuppressed mice of 5-6 weeks are commercially available. CD1 nu/nu mice are maintained under sterile conditions.

*Implant and growth of tumour cells*

Solid tumours from different hystotypes (lung, ovary, breast, prostate, pancreas, colon) can be transplanted subcutaneously (sc) in the axillary region of immunocompetent mice (murine models) or in immunodeprived (human models). Human tumours cell lines, originally obtained from ATCC, can be adapted to grow *in vivo* as solid tumours from "in vitro" cultures.

Murine or human haematological tumours models can be transplanted in different sites (ev, ip, ic o sc) of immunocompetent mice (murine tumours) or in immunodeprived mice (leukemia, lymphoma and human myeloma tumours).

*Pharmacological treatment*

Mice bearing solid or haematological tumours are randomized in experimental groups (10 mice/group). As far as solid tumours are concerned, a tumour mean weight of 80-100 mg for each group is required to start the treatment; mice with larger or smaller tumours are excluded.

The experimental groups are randomly allocated into the treatment group with the test substance or into the control group. The animals can be treated with the compounds through the intravenous, oral or intraperitoneal route, according to the bioavailability of the products and following different treatment protocols, for example iv once or twice a week or daily *per os.*

In solid tumour models, treatment with the compound can start when tumour weight is comprised between 80 and 100 mg after tumour transplant (day 0).

The compounds can be administered in a volume of 10 mL/Kg body weight /mouse in a suitable solvent.

*Antitumour activity parameters*

The following parameters can be determined for the evaluation of the antitumour activity:

- solid primary tumour growth, controlled twice a week in each mouse with a caliper;
- survival time of treated mice with respect to control mice;
- evaluation of the body weight of each mouse twice a week.

Inhibition of tumour growth, TWI% (percentage of inhibition of tumour growth compared to a control group treated with vehicle only), or relative inhibition of tumour growth RTWI% in case of advanced tumours is evaluated one week after treatment with the compound and tumour weight (TW) can be calculated as follows:

$$TW = 1/2\ ab^2$$

where a and b are the short and long diameters of the tumour mass expressed in millimiters.

Antitumour activity can be determined as inhibition of tumour growth (TWI %), calculated according to the following formula:

$$TWI\% = 100 - \frac{\text{mean TW (treated)}}{\text{mean TW (controls)}} \times 100$$

The relative percentage of inhibition of tumour growth compared to the control group treated with administration vehicle only (RTWI%) is evaluated one week after the last treatment with the product, according to the following formula:

$$RTWI\% = 100 - \frac{\text{mean RV of treated mice}}{\text{mean RV of control mice}} \times 100$$

where

$$RV = \frac{\text{Vt (tumour weight at t day)}}{\text{Vo (initial weight of the tumour at the beginning of the treatment)}}$$

The tumour regression percentage can be calculated as weight regressions of the tumour, determined as tumour weight at a certain day divided by the initial weight of the tumour at the beginning of the treatment.

In models of haematological tumours the antitumour activity can be determined as percentage increase of the mice mean survival time expressed as (T/C%) ratio between the mean survival time of the treated group (T) and the control group (C). Animals without tumours at the end of the experiment (60 days after tumour transplant) are excluded from calculation and are considered as long-term survivors (LTS).

*Toxicity evaluation in tumour-bearing mice*

Toxicity can be evaluated daily both on the basis of approximate autoptic results and of loss of body weight. When mice die before control animals treated with the vehicle only, or when a meaningful weight loss (>20%) and/or a reduction of spleen and liver dimensions is observed, it is assumed that death is due to toxicity.

The percentage change of body weight (BWC%) is calculated as follows: 100- (mean body weight of the mice at a given day / mean body weight of the mice at the beginning of the treatment) x 100. This value is calculated one week after treatment with the test compound.

## Claims

1. Compounds of formula (I)

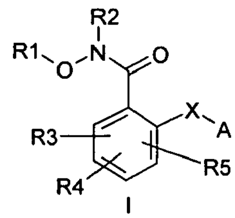

I

wherein:

R1 is selected from hydrogen, trifluoromethyl, $C_{1-10}$ alkyl, $C_{2-10}$ alkenyl, $C_{2-10}$ alkynyl, $C_{3-10}$ cycloalkyl, $C_{3-10}$ cycloalkylalkyl, aryl, aralkyl, heteroaryl, heteroarylalkyl, heterocyclyl, heterocycloalkyl, said alkyl, alkenyl, alkynyl, cycloalkyl, aryl, heteroaryl, and heterocycle groups being optionally substituted with one to three substituents independently selected from hydroxy, amino, alkyl or cycloalkyl;
R2 is selected from hydrogen, $C_{1-8}$ alkyl, CO-$C_{1-8}$ alkyl, aryl, aralkyl, or $C_{3-10}$ cycloalkyl, said alkyl, alkylcarbonyl, aryl, aralkyl or cycloalkyl groups being optionally substituted with one to three substituents independently selected from hydroxy, amino, alkyl or cycloalkyl;
R3, R4 and R5 are independently selected from hydrogen, hydroxy, halogen, trifluoromethyl, $C_{1-8}$ alkyl, $C_{1-8}$ alkoxy, nitro, cyano or $-(O)_m-(CH_2)_n-$R6 and $-(NH)_m-CH_2)_n-$R6 groups, wherein m is 0 or 1, R6 is hydrogen, hydroxy, carboxy or NRaRb wherein Ra and Rb are independently selected from hydrogen and $C_{1-4}$ alkyl, or, taken together with the nitrogen atom they are bound to, form a pyrrolidine, piperidine, morpholine or piperazine ring;
X is selected from O, S, SO, $SO_2$, NH, $CH_2$, CO;
A is an aromatic or aliphatic 5-6 membered heterocycle, containing from one to three nitrogen, oxygen or sulphur atoms, and optionally substituted on the carbon atoms with 1, 2 or 3 R7 groups or, on the nitrogen atoms, with $C_{1-10}$ alkyl, aryl, arylalkyl groups or with oxygen atoms to give N-oxides;
R7 is independently selected from hydrogen, $C_{1-20}$ alkyl, $C_{2-20}$ alkenyl, $C_{2-20}$ alkynyl, -OR7a, -SR7a, -CN, nitro, azido, halogen, haloalkyl, $-NH_2$, -NH($C_{1-10}$ alkyl), -N($C_{1-10}$ alkyl)$_2$, -NHC(=O)O-$C_{1-10}$ alkyl, -NHC(=O) $C_{1-10}$ alkyl, -COOH, -C(=O)O-$C_{1-10}$ alkyl, -C(=O)$C_{1-10}$ alkyl, -C(O)H, -S(=O)-$C_{1-10}$ alkyl, -S(=O)$_2$-$C_{1-10}$ alkyl, -S(=O)-aryl, -S(=O)$_2$-aryl, $C_{3-10}$ cycloalkyl optionally substituted with 1, 2 or 3 R8 groups, heterocyclyl optionally susbstituted with 1, 2 or 3 R8 groups;
R7a is selected from H, trifluoromethyl, $C_1$-$C_{20}$ alkyl, $C_2$-$C_{20}$ alkenyl, $C_2$-$C_{20}$ alkynyl, $C_{3-10}$ cycloalkyl, $C_{3-10}$ cycloalkylalkyl, aryl, arylalkyl, heteroaryl, heteroarylalkyl, heterocyclyl, heterocycloalkyl, said alkyl, alkenyl, alkynyl, cycloalkyl, aryl, heteroaryl, heterocycle groups being optionally substituted with one to three substituents independently selected from hydroxy, amino, alkyl or cycloalkyl;
R8 is independently selected from $C_{1-10}$ alkyl, $C_{2-10}$ alkenyl, $C_{2-10}$ alkynyl, phenyl, halogen, haloalkyl, alkoxy, thioalkoxy, amino, alkylamino, dialkylamino, carboxy, $C_{1-4}$ alkyl-OC(=O)-, $C_{1-4}$ alkyl-C(=O)-, aryl-OC(=O)-, $C_{1-4}$ alkyl-OC(=O)NH-, aryl-OC(=O)NH-, $C_{1-4}$ alkyl-C(=O)NH-, $C_{1-4}$ alkyl-C(=O)O-, ($C_{1-4}$ alkyl-O)$_r$- $C_{1-4}$ alkyl, HO-($C_{1-4}$ alkyl-O)$_r$- $C_{1-4}$ alkyl-, -OH, -SH, -CN, $-N_3$, -CNO, -CNS, $C_{1-4}$ alkyl-S(=O)-, $C_{1-4}$ alkyl-S(=O)$_2$-, $H_2$NS(=O)- and $H_2$NS(=O)$_2$-, where r is 1 or 2;

and enantiomers, diastereoisomers and pharmaceutically acceptable salts thereof.

**2.** Compounds according to claim 1 wherein R1 is selected from hydrogen, methyl, ethyl, tert-butyl, 2-hydroxyethyl, cyclopropylmethyl, 2-propenyl, carboxymethyl and (R)-2,3-dihydroxypropyl.

**3.** Compounds according to claim 1 or 2 wherein R2 is selected from hydrogen, methyl and ethyl.

**4.** Compounds according to any one of claims 1 - 3 wherein R2, R3 and R4 are independently selected from hydrogen, methyl, fluorine, chlorine, bromine and iodine.

**5.** Compounds according to any one of claims 1 to 4 wherein A is selected from pyrrole, furan, thiophene, pyrazole, thiazole, oxazole, imidazole, isothiazole, isoxazole, 1,2,3- triazole, 1,2,4-triazole, 1,2,4-oxadiazole, 1,3,4-oxadiazole, 1,2,5-oxadiazole, 1,2,5-thiadiazole, 1,3,4-thiadiazole, tetrazole, pyridine, pyrimidine, pyridazine, pyrazine, 1,2,4-triazine, 1,2,3-triazine, 1,2,4-triazine and 1,3,5-triazine.

**6.** Compounds according to claim 5 wherein A is selected from pyrrole, furan, thiophene and pyridine.

**7.** Compounds according to any one of claims 1 to 6 wherein R7 is selected from hydrogen, methyl, trifluoromethyl, fluorine, chlorine, bromine and iodine.

**8.** Compounds according to any one of claims 1 to 7 wherein X is NH and A is a pyridine ring.

**9.** Compounds according to any one of claims 1 to 7 wherein X is $-CH_2-$ or -CO- and A is a thiophene ring.

**10.** A compound selected from:

| | |
|---|---|
| | 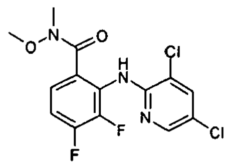 |
| | 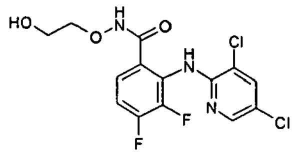 |

| | |
|---|---|
| | 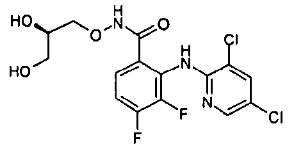 |
| | 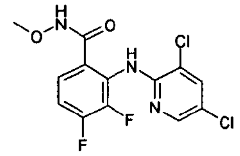 |
| | 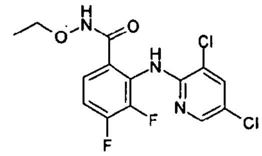 |
| | 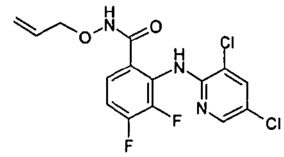 |
| | 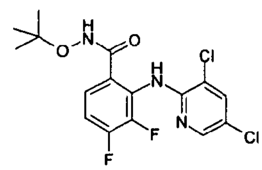 |
| | 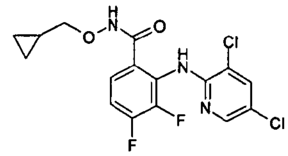 |
| | 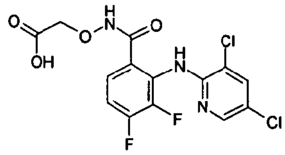 |
| | 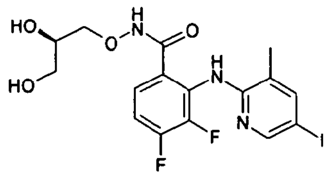 |
| | 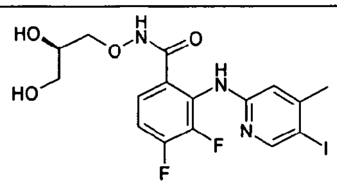 |
| | 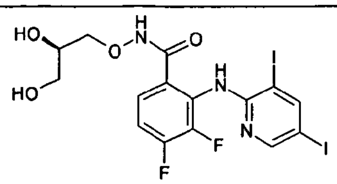 |

| | |
|---|---|
| | 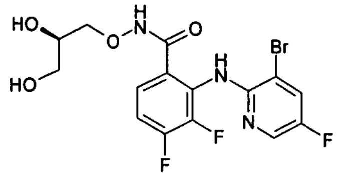 |
| | 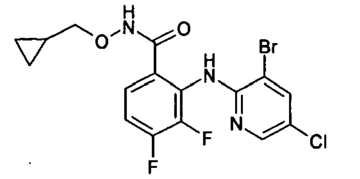 |
| | 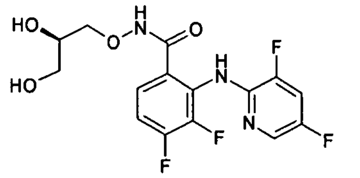 |
| | 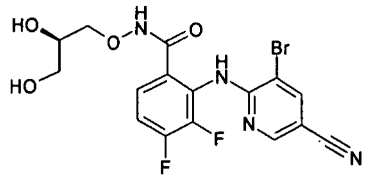 |
| | 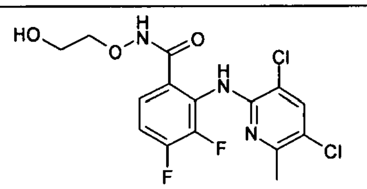 |
| | 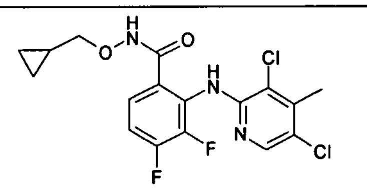 |
| | 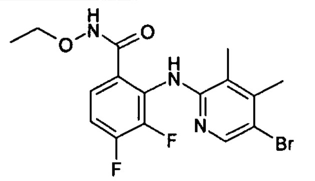 |
| | 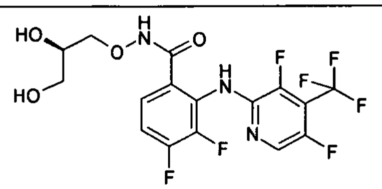 |
| | 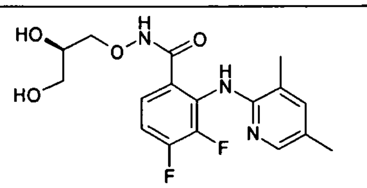 |

| | |
|---|---|
| | 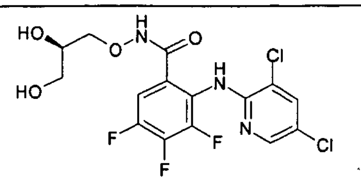 |
| | 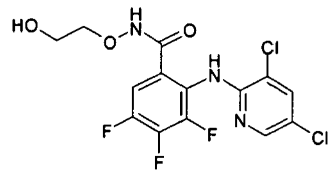 |
| | 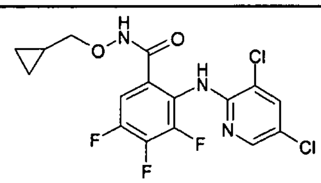 |
| | 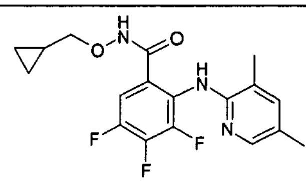 |
| | 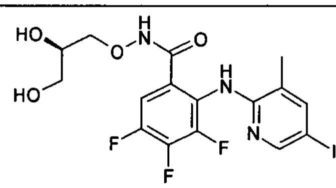 |
| | 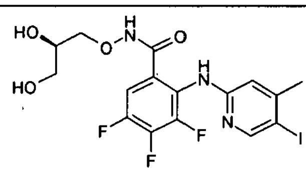 |
| | 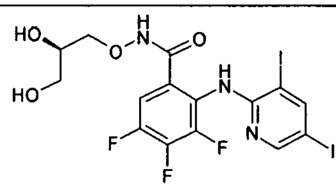 |
| | 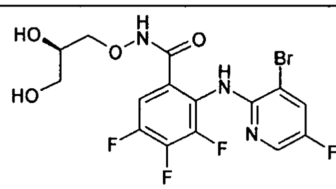 |
| | 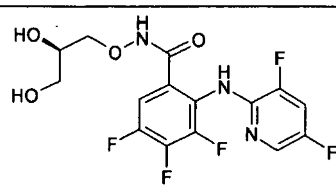 |

| | |
|---|---|
| | 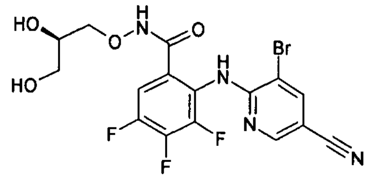 |
| | 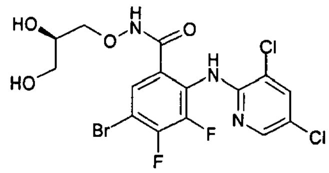 |
| | 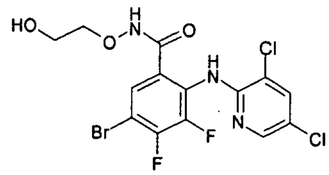 |
| | 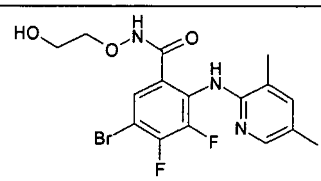 |
| | 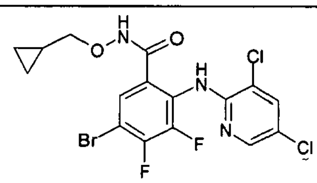 |
| | 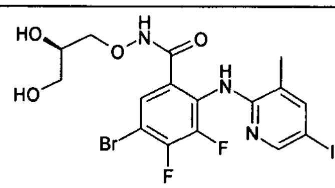 |
| | 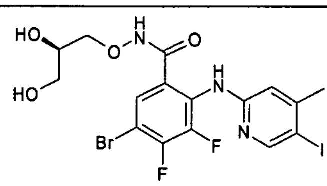 |
| | 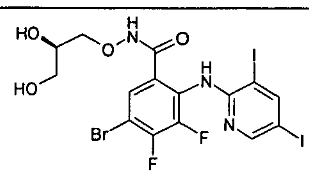 |
| | 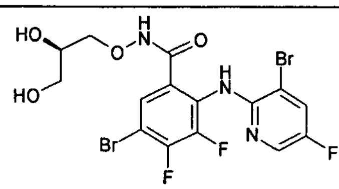 |

| | |
|---|---|
| | 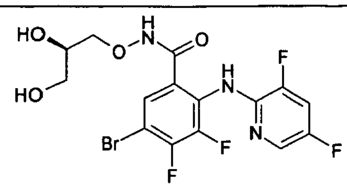 |
| | 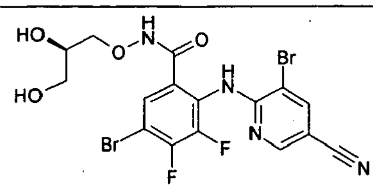 |
| | 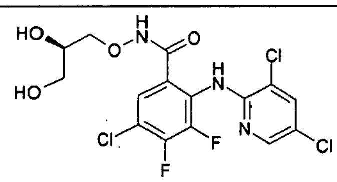 |
| | 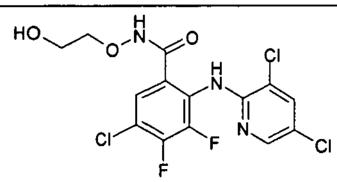 |
| | 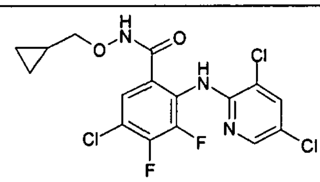 |
| | 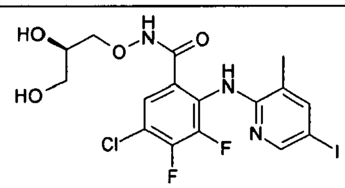 |
| | 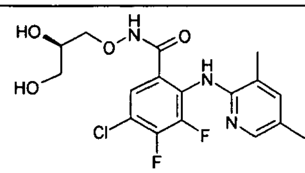 |
| | 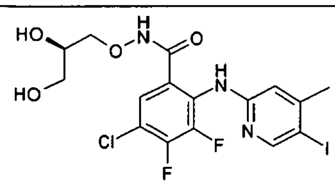 |
| | 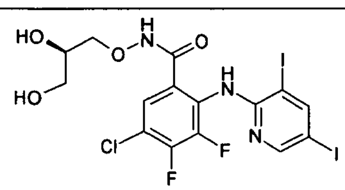 |

| | |
|---|---|
| | 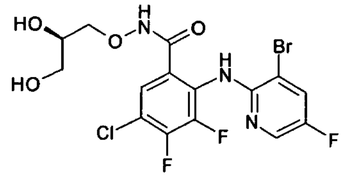 |
| | 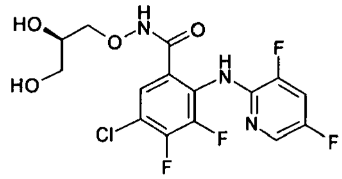 |
| | 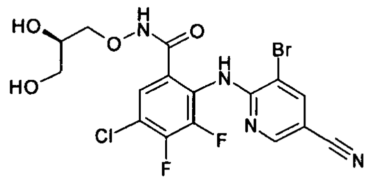 |
| | 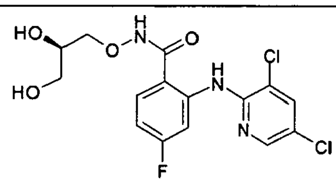 |
| | 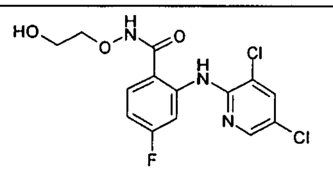 |
| | 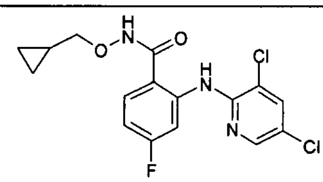 |
| | 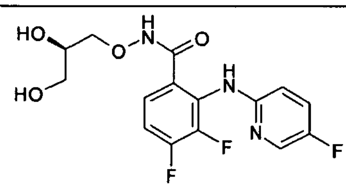 |
| | 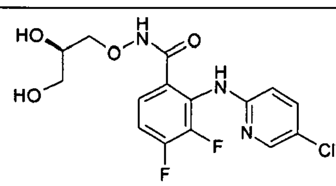 |
| | 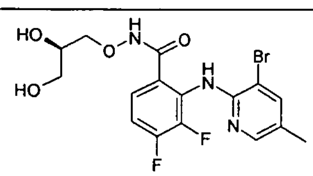 |

| | |
|---|---|
| | 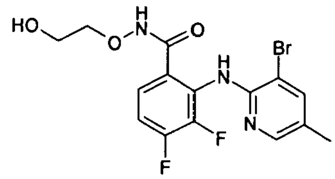 |
| | 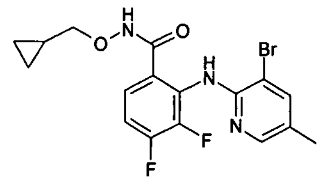 |
| | 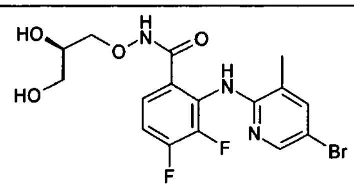 |
| | 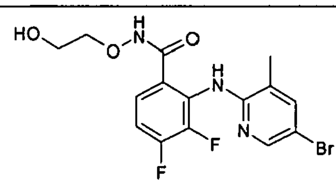 |
| | 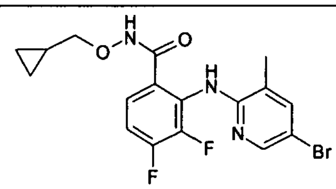 |
| | 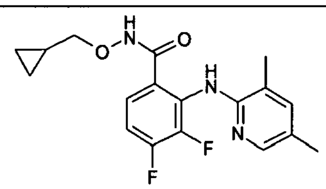 |
| | 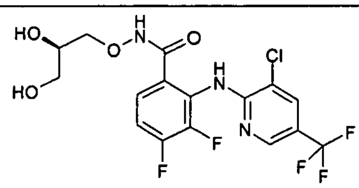 |
| | 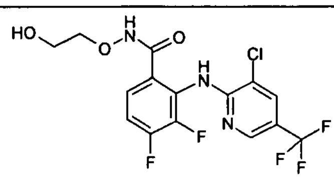 |
| | 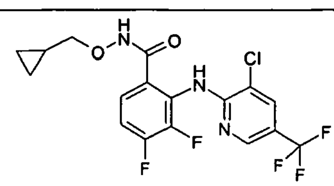 |

| | |
|---|---|
| | 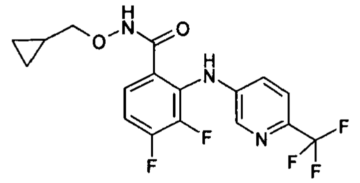 |
| | 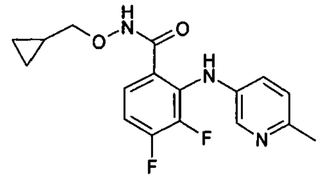 |
| | 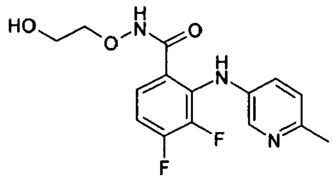 |
| | 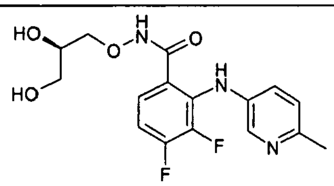 |
| | 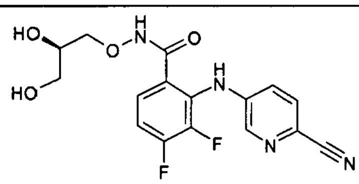 |
| | 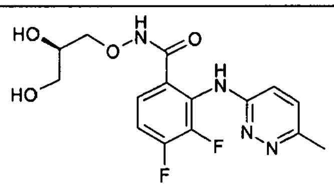 |
| | 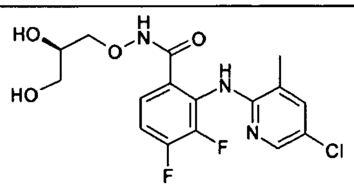 |
| | 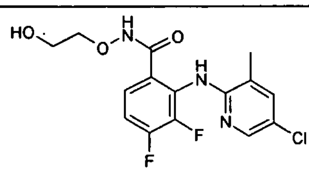 |
| | 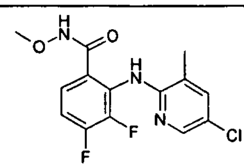 |

| | |
|---|---|
| | 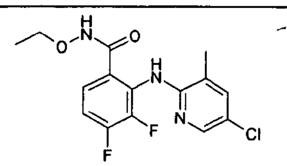 |
| | 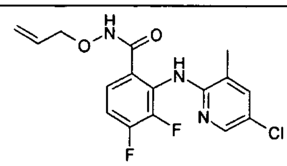 |
| | 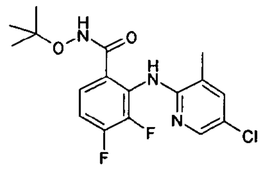 |
| | 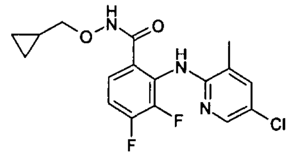 |
| | 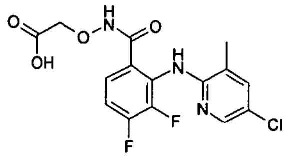 |
| | 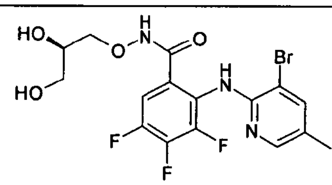 |
| | 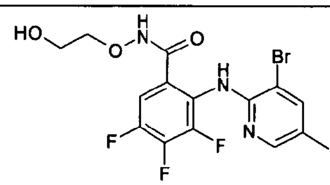 |
| | 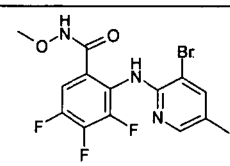 |
| | 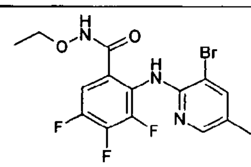 |
| | 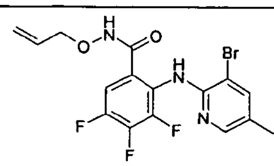 |

| | |
|---|---|
| | 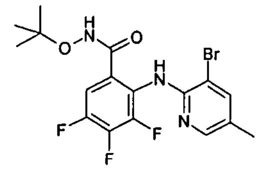 |
| | 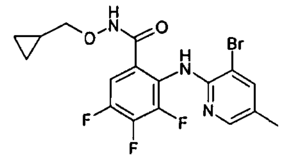 |
| | 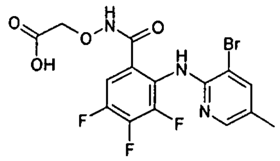 |
| | 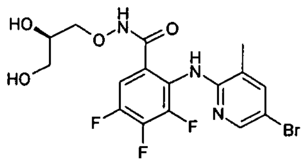 |
| | 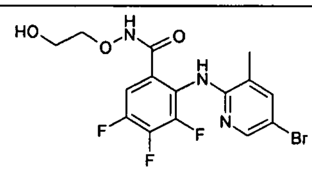 |
| | 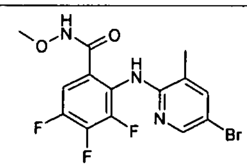 |
| | 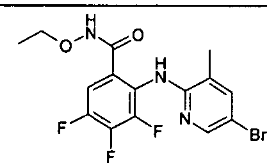 |
| | 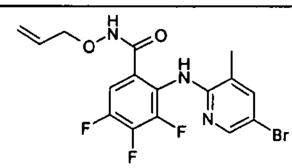 |
| | 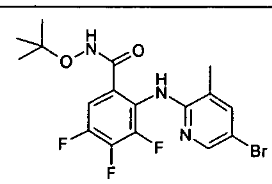 |
| | 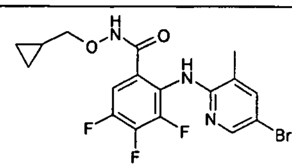 |

| | |
|---|---|
| | 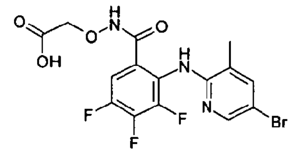 |
| | 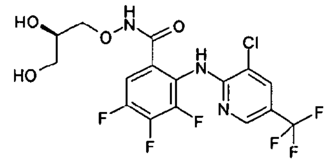 |
| | 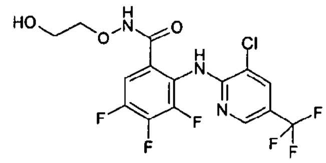 |
| | 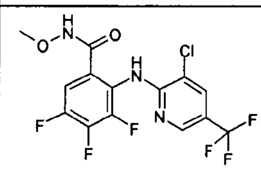 |
| | 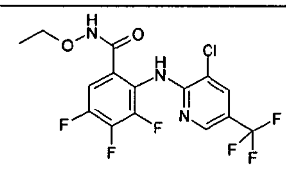 |
| | 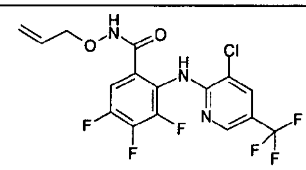 |
| | 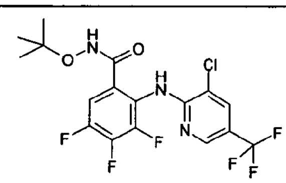 |
| | 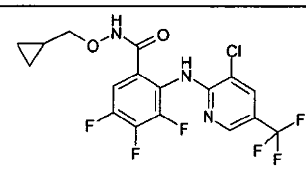 |
| | 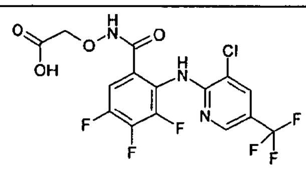 |
| | 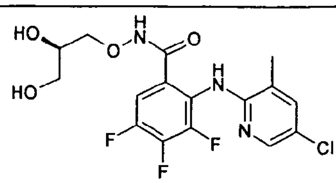 |

| | |
|---|---|
| | 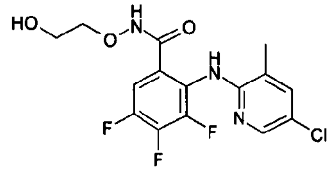 |
| | 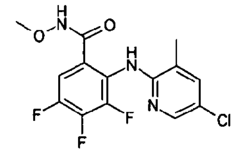 |
| | 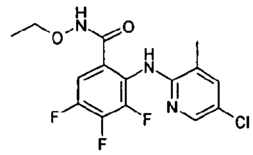 |
| | 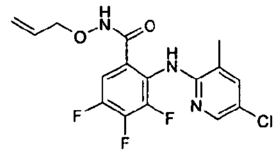 |
| | 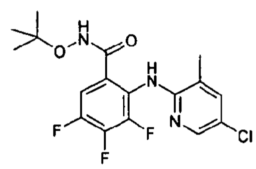 |
| | 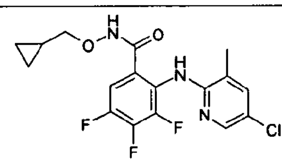 |
| | 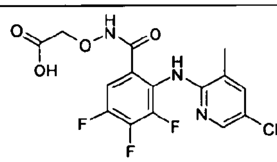 |
| | 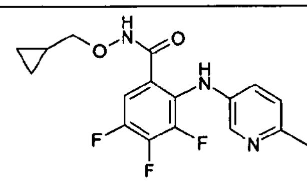 |
| | 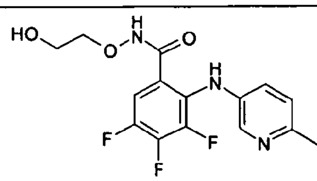 |
| | 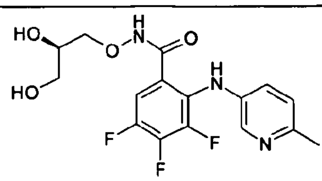 |

| | |
|---|---|
| | 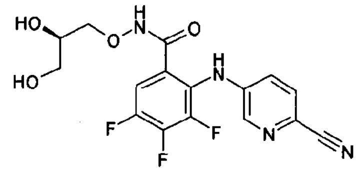 |
| | 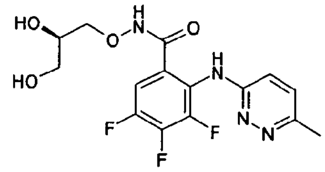 |
| | 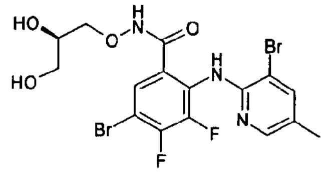 |
| | 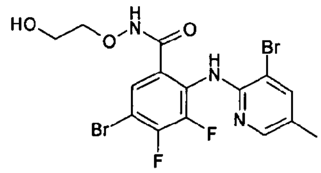 |
| | 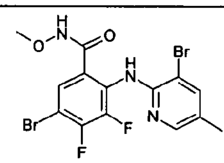 |
| | 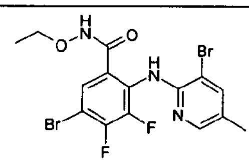 |
| | 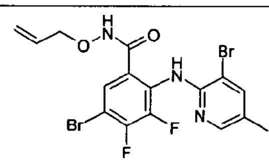 |
| | 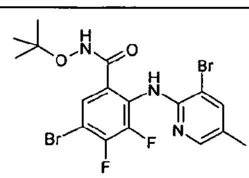 |
| | 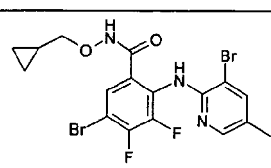 |
| | 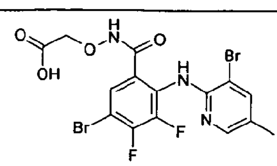 |

| | |
|---|---|
| | 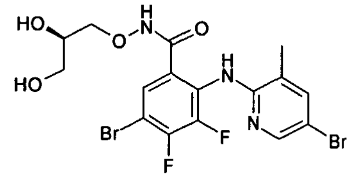 |
| | 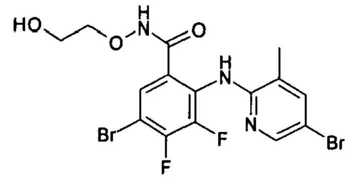 |
| | 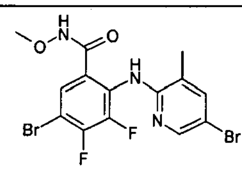 |
| | 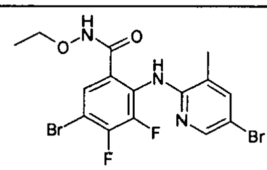 |
| | 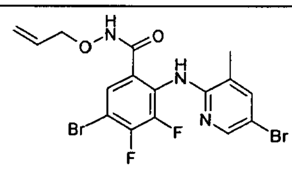 |
| | 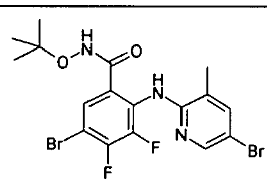 |
| | 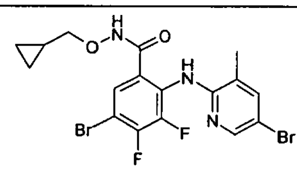 |
| | 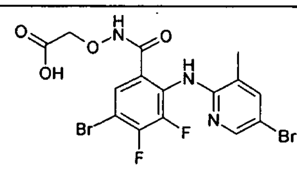 |
| | 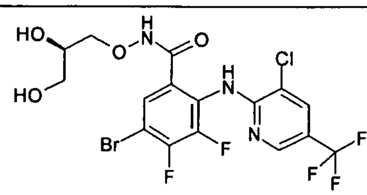 |

| | |
|---|---|
| | 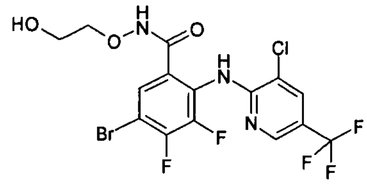 |
| | 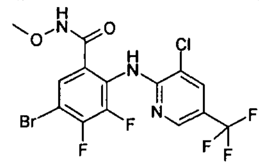 |
| | 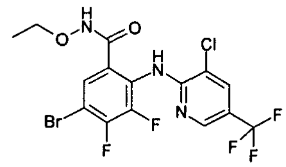 |
| | 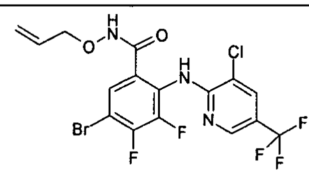 |
| | 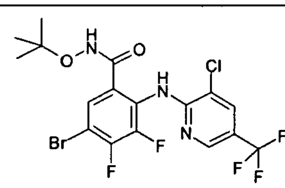 |
| | 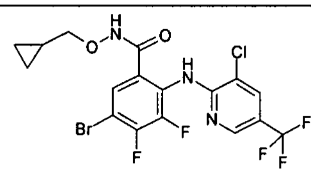 |
| | 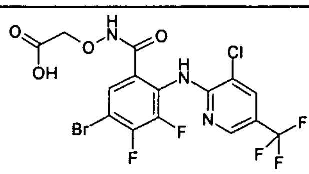 |
| | 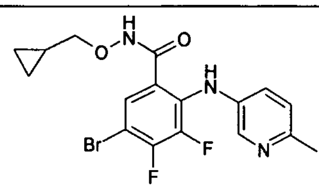 |
| | 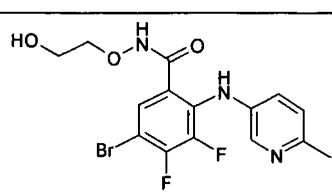 |

| | |
|---|---|
| | 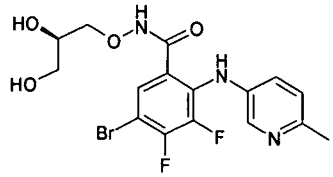 |
| | 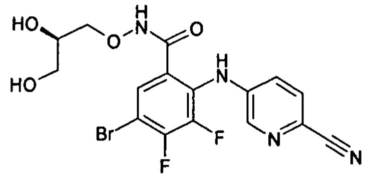 |
| | 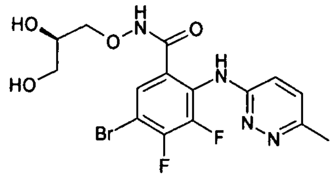 |
| | 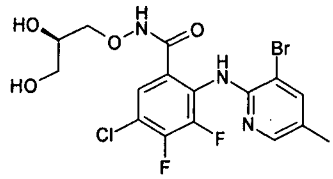 |
| | 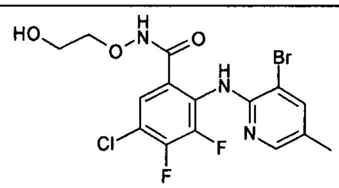 |
| | 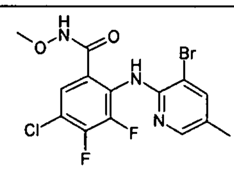 |
| | 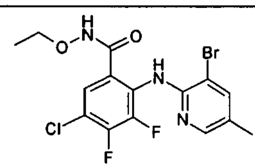 |
| | 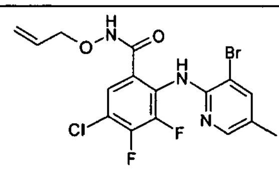 |
| | 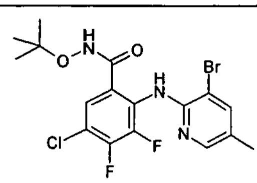 |

| | |
|---|---|
| | 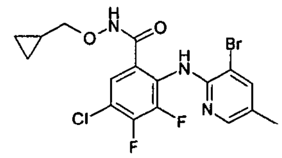 |
| | 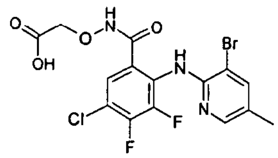 |
| | 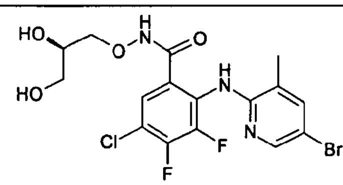 |
| | 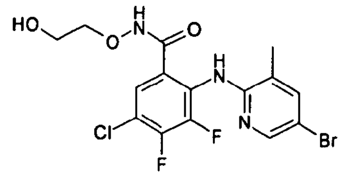 |
| | 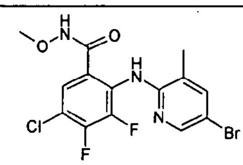 |
| | 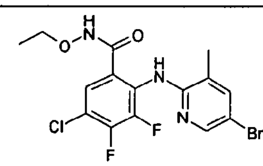 |
| | 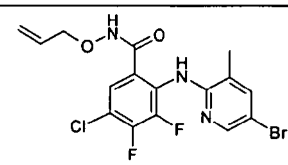 |
| | 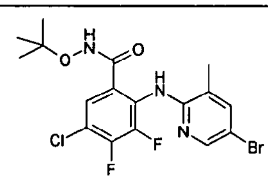 |
| | 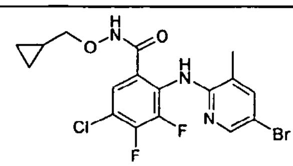 |
| | 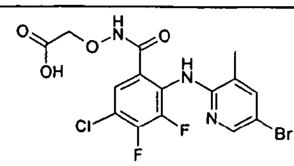 |

| | |
|---|---|
| | 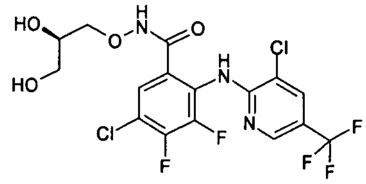 |
| | 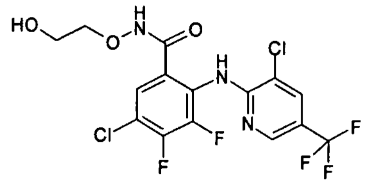 |
| | 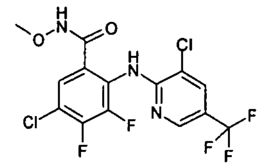 |
| | 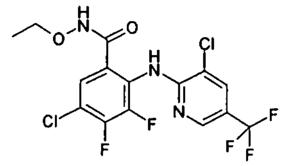 |
| | 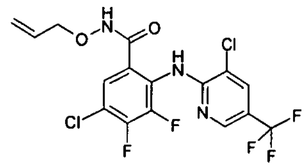 |
| | 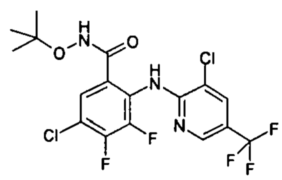 |
| | 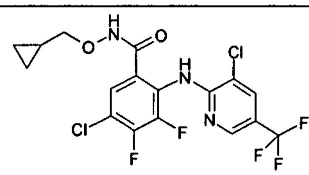 |
| | 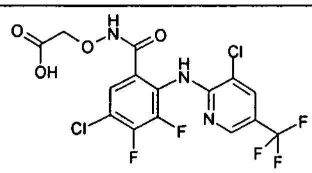 |
| | 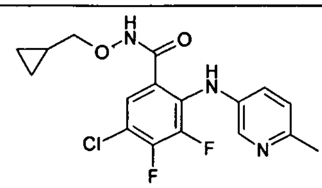 |

| | |
|---|---|
| | 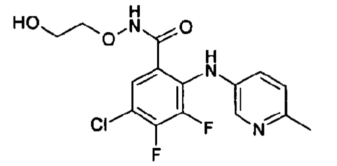 |
| | 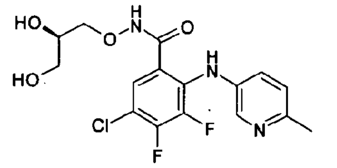 |
| | 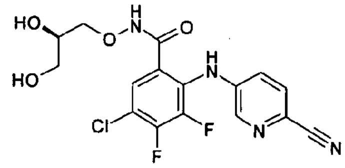 |
| | 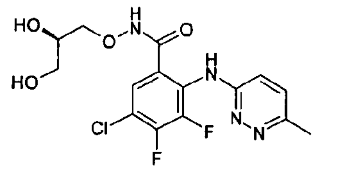 |
| | 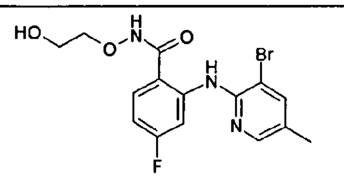 |
| | 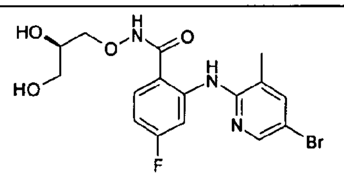 |
| | 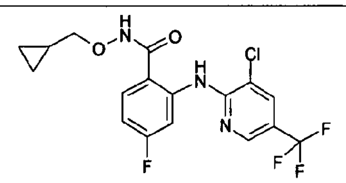 |
| | 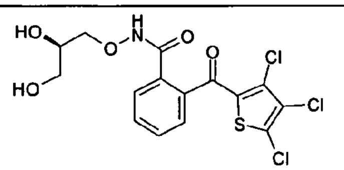 |
| | 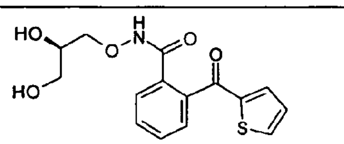 |
| | 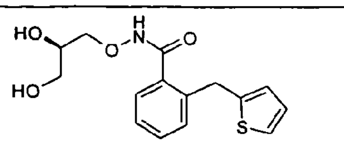 |

**11.** Compounds according to any one of claims 1 to 10 for use as medicaments.

**12.** Use of the compounds of any one of claims 1 to 10 for the preparation of pharmaceutical compositions for the treatment of hyperproliferative pathologies.

**13.** The use according to claim 12 wherein the hyperproliferative pathologies are brain tumours, lung, squamous cells, bladder, stomach, pancreas, breast, head, neck, kidney, ovary, prostate, colon-rectum, esophagus, testicles, thyroid or gynecological tumours.

**14.** Pharmaceutical compositions containing a compound of any one of claims 1 to 10 in admixture with suitable excipients or vehicles.

**15.** Pharmaceutical compositions according to claim 14 further containing a chemotherapeutic agent selected from mitosis inhibitors, alkylating agents, antimetabolites, intercalating agents, antibiotics, growth factors inhibitors, cell cycle inhibitors, topoisomerase inhibitors, anti-hormonal agents and angiogenesis agents.

European Patent Office

# EUROPEAN SEARCH REPORT

Application Number
EP 06 42 5676

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/007954 A2 (PHARMACIA CORP [US]; MCDONALD JOSEPH J [US]; BARTA THOMAS E [US]; BECK) 30 January 2003 (2003-01-30)<br>* tables *<br>* examples *<br>* paragraph [0098] - paragraph [0100] *<br>----- | 1-4,7, 11-15 | INV.<br>C07D213/74<br>C07D237/08<br>C07D333/28<br>C07D333/24<br>A61K31/44<br>A61K31/38<br>A61K31/50<br>A61P35/00 |
| X | WO 01/85680 A2 (PHARMACIA CORP [US]; BEDELL LOUIS J [US]; MCDONALD JOSEPH [US]; BARTA) 15 November 2001 (2001-11-15)<br>* tables *<br>* examples *<br>* page 26, paragraph 1 *<br>----- | 1-4,7, 11-15 | |
| X | WO 00/69819 A (SEARLE & CO [US]; BEDELL LOUIS J [US]; MCDONALD JOSEPH J [US]; BARTA T) 23 November 2000 (2000-11-23)<br>* tables *<br>* examples *<br>* page 25, last paragraph - page 26, paragraph 1 *<br>----- | 1-4,7, 11-15 | TECHNICAL FIELDS SEARCHED (IPC) |
| X | KASZTREINER, ENDRE ET AL: "Synthesis of O-substituted hydroxylamines" ACTA CHIMICA ACADEMIAE SCIENTIARUM HUNGARICAE , 84(2), 167-80 CODEN: ACASA2; ISSN: 0001-5407, 1975, XP008075212<br>* page 179, compound Vrc *<br>-----<br>-/-- | 1,3,4,7 | C07D<br>A61K<br>A61P |

The present search report has been drawn up for all claims

1

| Place of search | Date of completion of the search | Examiner |
|---|---|---|
| The Hague | 16 February 2007 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 42 5676

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | REGEL E ET AL: "C-ACYLIERUNG VON 5 GLIEDRIGEN N-HETEROCYCLEN, I ACYLIERUNG AN C-2 VON IMIDAZOLEN UND BENZIMIDAZOLEN C-ACYLATION OF 5-MEMBERED N-HETEROCYCLES, I. - ACYLATION AT C-2 OF IMIDAZOLES AND BENZIMIDAZOLES" JUSTUS LIEBIGS ANNALEN DER CHEMIE, VERLAG CHEMIE GMBH. WEINHEIM, DE, 1977, pages 145-158, XP009003467 ISSN: 0075-4617 * page 153; compound 1328 * ----- | 1-5,7 | |
| X | WO 93/24468 A (CIBA GEIGY AG [CH]; LUETHY CHRISTOPH [CH]; FISHER RAYMOND [GB]) 9 December 1993 (1993-12-09) * page 44 - page 45; table 3b * * page 52 - page 54 * ----- | 1-5,7,14 | |
| X | NEZU Y ET AL: "DIMETHOXYPYRIMIDINES AS NOVEL HERBICIDES. PART 2. SYNTHESIS AND HERBICIDAL ACTIVITY OF O-PYRIMIDINYLSALICYLATES AND ANALOGUES" PESTICIDE SCIENCE, ELSEVIER APPLIED SCIENCE PUBLISHER. BARKING, GB, vol. 47, no. 2, 1 June 1996 (1996-06-01), pages 115-124, XP000629092 ISSN: 0031-613X * page 118; compound 14 * ----- | 1-5,7 | TECHNICAL FIELDS SEARCHED (IPC) |
| D,A | WO 99/01426 A (WARNER LAMBERT CO [US]; BARRETT STEPHEN DOUGLAS [US]; BRIDGES ALEXANDE) 14 January 1999 (1999-01-14) * abstract * ----- | 1,10-12, 14 | |

The present search report has been drawn up for all claims

| Place of search | Date of completion of the search | Examiner |
|---|---|---|
| The Hague | 16 February 2007 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 06 42 5676

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2007

| Patent document cited in search report | Publication date | Patent family member(s) | Publication date |
|---|---|---|---|
| WO 03007954 A2 | 30-01-2003 | AU 2002326432 A1 | 03-03-2003 |
| | | BR 0211430 A | 13-07-2004 |
| | | CA 2453613 A1 | 30-01-2003 |
| | | EP 1406626 A2 | 14-04-2004 |
| | | JP 2005502632 T | 27-01-2005 |
| | | MX PA04000388 A | 07-03-2005 |
| WO 0185680 A2 | 15-11-2001 | AU 5958901 A | 20-11-2001 |
| WO 0069819 A | 23-11-2000 | AU 781339 B2 | 19-05-2005 |
| | | AU 4971800 A | 05-12-2000 |
| | | BR 0011291 A | 14-05-2002 |
| | | CA 2373500 A1 | 23-11-2000 |
| | | CN 1360577 A | 24-07-2002 |
| | | EP 1177173 A1 | 06-02-2002 |
| | | JP 2002544257 T | 24-12-2002 |
| | | MX PA01011481 A | 20-06-2005 |
| | | NZ 515197 A | 26-03-2004 |
| | | ZA 200109007 A | 31-01-2003 |
| WO 9324468 A | 09-12-1993 | AU 4314693 A | 30-12-1993 |
| | | MX 9303255 A1 | 31-01-1994 |
| | | ZA 9303815 A | 30-12-1993 |
| WO 9901426 A | 14-01-1999 | AT 277895 T | 15-10-2004 |
| | | AU 757046 B2 | 30-01-2003 |
| | | AU 8262798 A | 25-01-1999 |
| | | BR 9810366 A | 29-08-2000 |
| | | CA 2290506 A1 | 14-01-1999 |
| | | CN 1261877 A | 02-08-2000 |
| | | DE 69826662 D1 | 04-11-2004 |
| | | DE 69826662 T2 | 17-02-2005 |
| | | EP 0993439 A1 | 19-04-2000 |
| | | ES 2229515 T3 | 16-04-2005 |
| | | HR 980368 A2 | 30-04-1999 |
| | | HU 0003731 A2 | 28-04-2001 |
| | | IL 132840 A | 15-12-2004 |
| | | IS 5256 A | 19-11-1999 |
| | | JP 2002511092 T | 09-04-2002 |
| | | NO 996491 A | 29-12-1999 |
| | | NZ 501276 A | 27-10-2000 |
| | | PL 337698 A1 | 28-08-2000 |
| | | PT 993439 T | 31-12-2004 |
| | | TW 396149 B | 01-07-2000 |
| | | ZA 9805728 A | 27-01-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5525625 A
- WO 9837881 A
- WO 9901421 A
- WO 9901426 A
- WO 0040235 A
- WO 0040237 A
- WO 0041505 A
- WO 0041994 A
- WO 0042002 A
- WO 0042003 A
- WO 0042022 A
- WO 0042029 A
- WO 0056706 A
- WO 0068199 A
- WO 0068200 A
- WO 0068201 A
- WO 0105390 A
- WO 0105391 A
- WO 0105392 A
- WO 0105393 A
- WO 0168619 A
- WO 0176570 A
- WO 0206213 A
- WO 0206520 A
- WO 02069960 A
- WO 03035626 A
- WO 03047523 A
- WO 03053960 A
- WO 03077855 A
- WO 03077914 A
- WO 03103717 A
- WO 2004005284 A
- WO 2004045617 A
- WO 2004056789 A
- WO 2005000818 A
- WO 2005007616 A
- WO 2005009975 A
- WO 2005011466 A
- WO 2005023759 A
- WO 2005023251 A
- WO 2005028426 A
- WO 2005046665 A
- WO 2005051300 A
- WO 2005051301 A
- WO 2005051302 A
- WO 2005051906 A
- WO 2006056427 A
- WO 2006061712 A


### Non-patent literature cited in the description


- **HOSHINO et al.** *Oncogene,* 1999, vol. 18, 813-822
- **MANSOUR et al.** *Science,* 1994, vol. 265, 966-970
- **COWLEY et al.** *Cell,* 1994, vol. 77, 841-852
- **BRUNET et al.** *Oncogene,* 1994, vol. 9, 3379-3387
- **BOS, J.L.** *Cancer Res.,* 1989, vol. 49 (17), 4682-4689
- **DAVIES, H et al.** *Nature,* 2002, vol. 417 (6892), 949-954
- **COHEN, Y et al.** *Cancer Res.,* 2003, vol. 63 (15), 4561-4567
- **ANDERSON et al.** *Nature,* 1990, vol. 343, 651-653
- **SEGER et al.** *J. Biol. Chem.,* 1992, vol. 267, 14373-14381
- **SEBOLT-LEOPOLD et al.** *Nat. Med.,* 1999, vol. 5, 810-816
- **YEH, T. et al.** *Proc. Am. Assoc. Cancer Res.,* 2004, vol. 45
- **YEH, T et al.** *Proc. Am. Assoc. Cancer Res.,* 2004, vol. 45
- **LEE, P. et al.** *Proc. Am. Assoc. Cancer Res.,* 2004, vol. 45
- **WALLACE, E et al.** *Proceedings of the American Association of Cancer Research,* 2004, vol. 45
- **WINKLER, J.D et al.** *16th EORTC-NCI-AACR Symposium on Molecular Targets and Cancer Therapeutics,* 27 September 2004
- **TECLE, H et al.** *29th American Chemical Society Medicinal Chemistry Symposium,* 27 June 2004
- **KAUFMAN, M. D et al.** *Proc. Am. Assoc. Cancer Res.,* 2004, vol. 45
- **WATERHOUSE et al.** *Proceedings of the American Society for Clinical Oncology,* 2003, vol. 22
- **MOSMAN, T.** *J. Immunolog. Methods,* 1983, vol. 65, 66
- **GREEN, L.M.** *J. Immunol. Methods,* 1984, vol. 70, 257-268

- Remington's Pharmaceutical Sciences Handbook. Mack Pub